# EUROPEAN PATENT SPECIFICATION

(11) **EP 0 739 341 B1**
(45) Date of publication and mention of the grant of the patent: **02.06.1999**
(21) Application number: 95906329.8
(22) Date of filing: 11.01.1995
(51) Int. Cl.: C07D 401/04, A01N 43/54, C07D 401/14, C07D 405/04, C07D 239/24, C07D 417/04, C07D 409/04, C07D 405/14, C07D 417/14

(54) **HERBICIDAL ARYL AND HETEROARYL PYRIMIDINES**
ARYL- UND HETEROARYL- PYRIMIDINE ALS HERBIZIDE
PYRIMIDINES HERBICIDES D'ARYLE ET D'HETEROARYLE

(30) Priority: 12.01.1994 US 180257
(43) Date of publication of application: 30.10.1996
(73) Proprietor: Novartis AG, 4058 Basel (CH); Novartis-Erfindungen Verwaltungsgesellschaft m.b.H., 1230 Wien (AT)
(72) Inventor: BAUM, John, William, Palo Alto, CA 94303 (US); BAMBERG, Joe, Timothy, Palo Alto, CA 94301 (US); GRINA, Jonas, Antanas, Union City, CA 94587 (US)
(86) International application number: EP9500086
(87) International publication number: WO9519358

(56) References cited:
- EP-A- 0 484 750
- DE-A- 4 031 798
- US-A- 4 752 324
- BIOCHEM. INT., vol. 1982, 1982 pages 431-438, PRYOR ET AL 'Purification of maize alcohol dehydrogenase and competitive inhibition by pyrazoles.'
- AUST J.CHEM., vol. 32, 1979 pages 669-679, HARRIS ET AL 'Synthetic Plant Growth Regulators' cited in the application

## Description

### BACKGROUND OF THE INVENTION

This invention relates to novel substituted aryl and heteroaryl pyrimidines, their use as herbicides and agricultural compositions comprising the same.

Various pesticidal aryl and heteroaryl pyrimidines are known. U.S. Patent No. 4,752,324 discloses 2-(2-alkyl-6-arylpyrimidin-4-yl)nicotinic acid derivatives having herbicidal activity. DE 40 31 798 describes fungicidal substituted pyridylpyrimidines. Furthermore, Harris et al. Aust. J. Chem., 1979, 32, 669-679, describes the plant growth regulating properties of diaryl heterocyclic compounds.

### DESCRIPTION OF THE INVENTION

It has now been discovered that certain aryl and heteroaryl pyrimidines substituted at the 2 and 4-position of the pyrimidine ring exhibit herbicidal and plant growth regulating activity, when applied either pre or post emergence and used against annual and perennial grasses and broad leaf weeds.

The terms "herbicide" and "herbicidal" are used herein to denote the inhibitive control or modification of undesired plant growth. Inhibitive control and modification include all deviations from natural development such as, for example, total killing, growth retardation, defoliation, desiccation, regulation, stunting, tillering, stimulation, leaf burn, and dwarfing. The term "herbicidally effective amount" is used to denote any amount which achieves such control or modification when applied to the undesired plants themselves or to the area in which these plants are growing. The term "plants" is intended to include germinant seeds, emerging seedlings and established vegetation, including both roots and above-ground portions.

More particularly, this invention concerns compounds of the general formula I 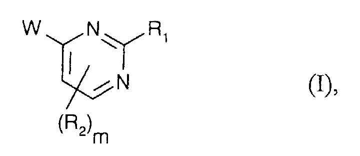 wherein
W is 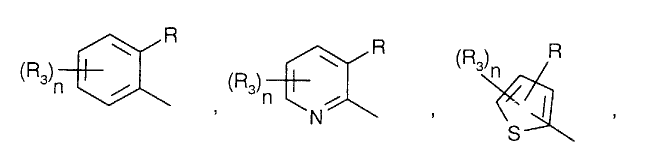 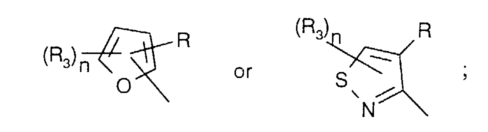
R³ is independently hydrogen, halogen, C₁-C₆-alkyl, C₁-C₆-haloalkyl, C₁-C₆-alkoxy, cyano, C₁-C₆-alkoxycarbonyl, C₁-C₆-alkylamino, di-C₁-C₆-alkylamino or -N(R⁵)-CO-R⁶;
n is is to 4;
R is CO₂R⁴, CHO, CONH-O-CH₂CO₂R⁴, COSR⁴, CO₂CHR⁵OCOR⁶ or CH=N-OR⁴;
R¹ is Ar, (Z)ₓY-Ar, or ZAr wherein Ar is an optionally substituted aryl or hetero aryl group selected from the group consisting of phenyl, pyridyl, piperonyl, naphthyl, indolyl, quinolyl, isoquinolyl, quinoxalinyl, quinazolinyl, benzoxazolyl, benzothiazolyl, phenanthryl, pyridyl-N-oxide, anthranilyl, pyrimidinyl, pyrazinyl, thienyl, furyl, pyrrolyl, oxazolyl, thiazolyl, isoxazolyl, isothiazolyl, imidazolyl, pyrazolyl, oxadiazolyl and thiadiazolyl wherein the optional substituents are phenoxy, halo, C₁-C₆-alkyl, C₂-C₆-alkenyl, C₁-C₆-haloalkyl, C₁-C₆-haloalkylthio, C₁-C₆-alkylthio, C₁-C₆-alkylsulfinyl, C₁-C₆-alkylsulfonyl C₁-C₆-alkoxy, C₁-C₆-haloalkoxy, C₁-C₆-alkoxy-C₁-C₆-alkyl, cyano, nitro, C₁-C₆-alkoxycarbonyl, amino, C₁-C₆-alkylamino, di-C₁-C₆-alkylamino, hydroxy, Y is O, S or NH; Z is an optionally substituted C₁-C₃alkyl, C₂-C₄alkynyl or an optionally substituted C₂-C₄alkenyl, wherein the substituents are independently C₁-C₆-alkyl and halogen, x is 0 to 2;
R² is independently hydrogen, halogen, C₁-C₆-alkyl, C₂-C₆-alkenyl, C₁-C₆-haloalkyl, C₁-C₆-alkylthio, C₁-C₆-alkylsulfinyl, C₁-C₆-alkylsulfonyl, C₁-C₆-alkoxy, C₁-C₆-alkoxy-C₁-C₆-alkyl, cyano, nitro, amino, C₁-C₆-alkylamino, di-C₁-C₆-alkylamino, CO₂R⁴ or hydroxy, m is 1 to 2;
R⁴ is hydrogen, alkali or alkaline earth cation, ammonium cation, substituted ammonium cation, phosphonium cation, C₁-C₆-alkyl phosphonium cation, tri-C₁-C₆-alkylsulfonium cation, tri-C₁-C₆-alkylsulfoxonium cation, C₁-C₆-alkyl, C₂-C₆-alkenyl, C₁-C₆-haloalkyl, C₁-C₆-alkoxy-C₁-C₆-alkyl, optionally substituted phenyl or optionally substituted phenyl-C₁-C₆-alkyl;
R⁵ is hydrogen or C₁-C₆-alkyl ; and
R⁶ is C₁-C₆-alkyl, C₂-C₆-alkenyl, C₁-C₆-haloalkyl, C₁-C₆-alkoxy-C₁-C₆-alkyl, optionally substituted phenyl and optionally substituted phenyl-C₁-C₆-alkyl, wherein the optional substituents are C₁-C₆-alkyl, C₁-C₆-haloalkyl, halogen, C₁-C₆-alkoxy, C₂-C₆-alkenyl, cyano and nitro;
provided that (i) when R¹ is phenyl; W is not 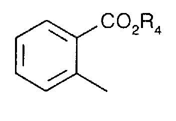 and (ii) when R¹ is optionally substituted phenyl and W is 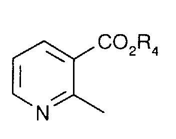 R² is not C₁-C₆-alkyl or C₂-C₆-alkenyl.

The term "alkyl" as used herein includes straight, branched and cyclo alkyl groups, preferably containing up to 6 carbon atoms. This applies to alkyl moieties contained for example, in "haloalkyl" and each alkyl group of "alkoxyalkyl". The term "alkenyl is represented by 2 to 6 carbon atoms.

Suitable halogen groups include fluorine, chlorine, bromine, and iodine. Haloalkyl groups may be substituted by one or more halogen atoms. The term "alkali cation" is defined as metals of group 1A of the periodic chart and particularly include sodium and potassium. The term "alkaline earth cation" includes magnesium, calcium, strontium and barium.

The term "phenylalkyl" refers to an alkyl group substituted with a phenyl. The terms "optionally substituted phenyl", "optionally substituted phenylalkyl" and "optionally substituted phenoxy" refers to a phenyl, phenylalkyl, or phenoxy group substituted at one or more of the ring carbon atoms with a group selected from alkyl, haloalkyl, halogen, alkoxy, alkenyl, cyano, and nitro.

The term "substituted ammonium cation" refers to an ammonium cation substituted by a C₁-C₂₀alkyl, di-C₁-C₂₀alkyl, tri-C₁-C₂₀alkyl, tetra-C₁-C₂₀alkyl, hydroxy-C₁-C₅alkyl, di(hydroxy-C₁-C₅alkyl), tri(hydroxy-C₁-C₅alkyl), C₁₋₅alkoxyC₁-C₅alkyl, hydroxy-C₁-C₅alkoxy-C₁-C₅alkyl or C₁-C₅alkoxycarbonyl-C₁-C₅alkyl group.

A particularly preferred subgroup of compounds of formula I are compounds wherein
(i) W is 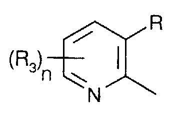
(ii) R is CO₂R⁴, CHO, CONH-O-CH₂CO₂R⁴, COSR⁴, CO₂CHR⁵OCOR⁶ or CH=NOR⁴;
(iii) R¹ is Ar, (Z)ₓYAr or ZAr wherein Ar is an optionally substituted phenyl, pyridyl, naphthyl, indolyl, piperonyl, quinolyl, benzoxazolyl, benzothiazolyl, anthranilyl or phenanthryl; Y is O, S or NH; Z is an optionally substituted C₁-C₃alkyl, C₂-C₄alkynyl or optionally substituted C₂-C₄alkenyl wherein the substituents are independently alkyl and halogen and x is 0 to 2;
(iv) R² is hydrogen, CO₂R⁴ or C₁-C₆-alkoxy;
(v) R³ is hydrogen or halogen.
(vi) R⁴ is hydrogen, alkali or alkaline earth cation; ammonium cation, substituted ammonium cation, phosphonium cation, C₁-C₆-alkyl phosphonium cation, tri-C₁-C₆-alkylsulfonium cation, tri-C₁-C₆-alkylsulfoxonium cation, C₁-C₆-alkyl, C₂-C₆-alkenyl, C₁-C₆-haloalkyl, C₁-C₆-alkoxy-C₁-C₆-alkyl, optionally substituted phenyl or optionally substituted phenyl-C₁-C₆-alkyl;
(vii) R⁵ is hydrogen or C₁-C₆-alkyl; and
(viii) R⁶ is C₁-C₆-alkyl, C₂-C₆-alkenyl, C₁-C₆-haloalkyl, C₁-C₆-alkoxy- C₁-C₆-alkyl, optionally substituted phenyl or optionally substituted phenyl-C₁-C₆-alkyl.

Preferably R is CO₂CHR⁵OCOR⁶ or CO₂R⁴ wherein R⁴ is hydrogen, C₁-C₆-alkyl, alkali or alkaline earth cations, ammonium cation, substituted ammonium cation, phosphonium cation, C₁-C₆-alkyl phosphonium cation, tri-C₁-C₆-alkylsulfonium cation, or tri-C₁-C₆-alkyl sulfoxonium cation and
R¹ is optionally substituted phenyl, pyridyl, naphthyl, quinolyl, piperonyl, (Z)ₓOphenyl and (Z)phenyl wherein Z is C₁-C₃alkyl, C₂-C₄alkynyl or C₂-C₄alkenyl and x is 1.

More prefreably R is CO₂R⁴ wherein R⁴ is hydrogen, Na, NH₄, K, Ca, Mg, trimethyl sulfonium, trimethyl sulfoxonium or isopropyl ammonium; and
R¹ is phenyl or substituted phenyl wherein the substituents are independently methyl, methoxy, chloro, fluoro, amino, C₁-C₆-haloalkoxy, nitro or C₁-C₆-haloalkyl; and
R² is hydrogen, C₁-C₆-alkoxy, or CO₂R⁴.

Another particularly preferred subgroup of compounds of formula I are compounds wherein
(i) W is 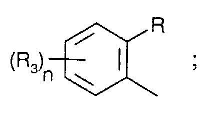
(ii) R is CO₂R⁴, CHO, CONH-O-CH₂CO₂R⁴, COSR⁴, CO₂CHR⁵OCOR⁶ or CH=NOR⁴;
(iii) R¹ is Ar, (Z)ₓYAr or ZAr wherein Ar is an optionally substituted phenyl, pyridyl, naphthyl, indolyl; piperonyl, quinolyl, benzoxazolyl, benzothiazolyl, anthranilyl or phenanthryl; Y is O, S or NH; Z is an optionally substituted C₁-C₃alkyl, C₂-C₄alkynyl or optionally substituted C₂-C₄alkenyl wherein the substituents are independently alkyl and halogen and x is 0 to 2;
(iv) R² is hydrogen, CO₂R⁴, C₁-C₆-alkoxy or C₁-C₆-alkyl;
(v) R³ is hydrogen or halogen and n is 1 or 2;
(vi) R⁴ is hydrogen, alkali or alkaline earth cation, ammonium cation, substituted ammonium cation, phosphonium cation, C₁-C₆-alkyl phosphonium cation, tri-C₁-C₆-alkylsulfonium cation, tri-C₁-C₆-alkylsulfoxonium cation, C₁-C₆-alkyl, C₂-C₆-alkenyl, C₁-C₆-haloalkyl, C₁-C₆-alkoxy-C₁-C₆-alkyl, optionally substituted phenyl or optionally substituted phenyl-C₁-C₆-alkyl;
(vii) R⁵ is hydrogen or C₁-C₆-alkyl; and
(viii) R⁶ is C₁-C₆-alkyl, C₂-C₆-alkenyl, C₁-C₆-haloalkyl, C₁-C₆-alkoxy-C₁-C₆-alkyl, optionally substituted phenyl or optionally substituted phenyl-C₁-C₆-alkyl.

Preferably R is CO₂CHR⁵OCOR⁶ or CO₂R⁴ wherein R⁴ is hydrogen, C₁-C₆-alkyl, alkali or alkaline earth cations, ammonium cation, substituted ammonium cation, phosphonium cation, C₁-C₆-alkyl phosphonium cation, tri-C₁-C₆-alkylsulfonium cation, or tri-C₁-C₆-alkyl sulfoxonium cation and
R¹ is optionally substituted phenyl, pyridyl, naphthyl, quinolyl, piperonyl, (Z)ₓOphenyl and (Z)phenyl wherein Z is C₁-C₃alkyl, C₂-C₄alkynyl or C₂-C₄alkenyl and x is 1.

More preferably R is CO₂R⁴ wherein R⁴ is hydrogen, Na, NH₄, K, Ca, Mg, trimethyl sulfonium, trimethyl sulfoxonium or isopropyl ammonium; and
R¹ is phenyl or substituted phenyl wherein the substituents are independently methyl, methoxy, chloro, fluoro, amino, C₁-C₆-haloalkoxy, nitro or C₁-C₆-haloalkyl and R³ is halogen.

Another preferred sub group of compounds of Formula I include the compounds wherein R is CO₂R⁴; R⁴ is hydrogen, Na, NH₄, K, Ca, Mg, trimethylsulfonium, trimethylsulfoxonium or isopropylammonium; R¹ is Ar, (Z)ₓY-Ar or ZAr wherein Ar is an optionally substituted phenyl, pyridyl, naphthyl, indolyl, piperonyl, quinolyl, benzoxazolyl, benzothiazolyl or phenanthryl wherein the optional substituents are halo, C₁-C₆-alkyl, C₂-C₆-alkenyl, C₁-C₆-haloalkyl, C₁-C₆-alkylthio, C₁-C₆-alkylsulfinyl, C₁-C₆-alkylsulfonyl, C₁-C₆-alkoxy, C₁-C₆-haloalkoxy, C₁-C₆-alkoxy-C₁-C₆-alkyl, cyano, nitro, C₁-C₆-alkoxycarbonyl, amino, C₁-C₆-alkylarnino, di-C₁-C₆-alkylamino or hydroxy; Y is O, S or NH, preferably O; Z is an optionally substituted C₁-C₃alkyl, C₂-C₄alkynyl or an optionally substituted C₂-C₄alkenyl wherein the substituents are independently C₁-C₆-alkyl and halogen; and x is 0 to 2.

Still another preferred subgroup of compounds of Formula I include the compounds wherein
W is 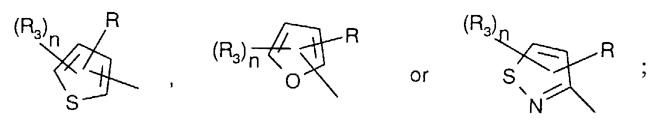
R is CO₂R⁴; CHO; CONH-O-CH₂CO₂R⁴; COSR⁴; CO₂CHR⁵OCOR⁶ or CH=NOR⁴;
R¹ is Ar, ZₓOAr or ZAr wherein Ar is an optionally substituted phenyl, pyridyl, naphthyl, indolyl, piperonyl, quinolyl, benzoxazolyl, benzothiazolyl or phenanthryl;
R² is hydrogen; and
R³ is hydrogen or halogen.
A further preferred subgroup of compounds of Formula I includes the compounds wherein R is CO₂R⁴; R⁴ is hydrogen, Na, NH₄; K, Ca, Mg, trimethylsulfonium, trimethylsulfoxonium or isopropylammonium; R¹ is Ar, (Z)ₓY-Ar or Zar wherein Ar is an optionally substituted phenyl, pyridyl, naphthyl, indolyl, piperonyl, quinolyl, benzoxazolyl, benzothiazolyl, anthranilyl or phenanthryl wherein the optional substituents are halo, C₁-C₆-alkyl, C₂-C₆-alkenyl, C₁-C₆-haloalkyl, C₁-C₆-alkylthio, C₁-C₆-alkylsulfinyl; C₁-C₆-alkylsulfonyl, C₁-C₆-alkoxy, C₁-C₆-haloalkoxy C₁-C₆-alkoxy-C₁-C₆-alkyl, cyano, nitro, C₁-C₆-alkoxycarbonyl, amino, C₁-C₆-alkylarnino, di-C₁-C₆-alkylamino or hydroxy; Y is O, S or NH; Z is an optionally substituted C₁-C₃-alkyl, C₂-C₄-alkynyl, or an optionally substituted C₂-C₄-alkenyl wherein the substituents are independently C₁-C₆-alkyl and halogen; and X is 0 to 2.

In another embodiment the invention includes a herbicidal composition comprising a herbicidally effective amount of a compound of Claim 1 in association with an agriculturally acceptable diluent.

A general process for making the compounds of this invention is as follows.

Appropriately substituted 1-(aryl or heteroaryl)-3-(N,N-dimethylamino)prop-2-en-1-one is heated with an appropriately substituted amidine or amidine hydrochloride and a (optional) base, such as sodium methoxide, in a suitable solvent, such as methanol, to afford the substituted 4-(aryl or heteroaryl)pyrimidine.

The substituted amidines used in this process were either purchased or prepared from commercially available starting materials. For example, the procedure of R.S.Garigipati *(Tetrahedron Letters,* 31, 1969 (1990)) was used to prepare amidines from the appropriately substituted nitrile by reaction with chloromethylaluminum amide in toluene.

The process for making the compounds of this invention will be more fully understood by reference to the following examples.

### EXAMPLE 1

### a) Preparation of 1-(3-methoxcarbonylpyridin-2-yl)-3-(N,N-dimethylamino)prop-2-en-1-one.

A suspension of 27.1 g (164 mmol) 2-acetylpyridine-3-carboxylic acid in 200 mL toluene is heated to reflux to remove water by azeotropic distillation. After approximately 20 mL of distillate is collected, the solution is cooled to ambient temperature and 54 mL N,N-dimethylformamide dimethylacetal is added dropwise. The brown solution is heated to reflux for 5 h, allowed to cool and concentrated to low volume *in vacuo.* This solution is treated with diethyl ether and stirred overnight. The orange crystals (mp 126-129°C) are collected by vacuum filtration. The ¹H NMR and mass spectra are consistent with 1-(3-methoxycarbonylpyridin-2-yl)-3-(N,N-dimethylamino)prop-2-en-1-one.

### b) Preparation of 2-[4-(2-phenyl)pyrimidinyl]-3-pyridinecarboxylic acid (Compound 7 in Table 1).

To a solution of 2.00 g (8.54 mmol) 1-(3-methoxycarbonylpyridin-2-yl)-3-(N,N-dimethylamino)prop-2-en-1-one and 1.50 g (8.54 mmol) benzamidine hydrochloride hydrate in 100 mL anhydrous methanol, is added 4.0 mL 25% sodium methoxide in methanol. The resulting dark solution is refluxed for 6 h allowing about half of the methanol to distill off, then stirred at ambient temperature for 72 h and evaporated to dryness *in vacuo.* The residue is partitioned between ethyl acetate and 0.5 M aqueous sodium hydroxide. The aqueous layer is washed once with ethyl acetate and acidified to pH 3 with concentrated HCl. The solid precipitate is collected by vacuum filtration and dried *in vacuo* at 50°C for 1 h to yield a tan solid, m.p. 169-170°C. The ¹H NMR and mass spectra are consistent with the desired 2-[4-(2-phenyl)pyrimidinyl]-3-pyridinecarboxylic acid.

### c) Preparation of 2-[4-(2-phenyl)pyrimidinyl]-3-pyridinecarboxylic acid, monosodium salt (Compound 1 is in Table 1).

To a slurry of 1.00 g (3.61 mmol) 2-[4-(2-phenyl)pyrimidinyl]-3-pyridinecarboxylic acid in 10 mL methanol, is added 0.82 mL (3.61 mmol) 25% sodium methoxide in methanol. The solution is stirred for 5 min and evaporated *in vacuo* to yield a solid foam. The ¹H NMR and mass spectra are consistent with the 2-[4-(2-phenyl)pyrimidinyl]-3-pyridinecarboxylic acid, monosodium salt.

### EXAMPLE 2

### a) Preparation of 2-[4-[2-(3-chlorophenyl)]pyrimidinyl]-3-pyridinecarboxylic acid (Compound 14 in Table 1).

To a solution of 4.29 g (18.32 mmol) 1-(3-methoxycarbonylpyridin-2-yl)-3-(N,N-dimethylamino)prop-2-en-1-one and 3.50 g (18.32 mmol) 3-chlorobenzamidine hydrochloride in 150 mL methanol, is added 8.5 mL 25% sodium methoxide in methanol. The resulting brown solution is refluxed for approximately 6 h allowing about half of the methanol to distill off, then allowed to cool, and evaporated *in vacuo.* The residue is partitioned between water and ethyl acetate. An insoluble solid is collected by vacuum filtration. This solid is dissolved in 1 M aqueous sodium hydroxide and acidified to pH 3 with concentrated HCl. The resulting precipitate is collected by vaccum filtration and dried to yield a tan solid (mp 163-166°C). The ¹H NMR and mass spectra are consistent with those expected for 2-[4-[2-(3-chlorophenyl)]pyrimidinyl]-3-pyridinecarboxylic acid.

### EXAMPLE 3

### Preparation of 2-[4-[2-(4-fluorophenyl)]pyrimidinyl]-3-pyridinecarboxylic acid (Compound 16 in Table 1).

To a solution of 5.88 g (25.1 mmol) 1-(3-methoxycarbonylpyridin-2-yl)-3-(N,N-dimethylamino)prop-2-en-1-one and 3.47 g (25.1mmol) 4-fluorobenzamidine in 150 mL methanol is added 12.1 mL (53 mmol) 25% sodium methoxide in methanol. The solution is stirred and heated to reflux allowing some of the methanol to distill off. After 2 hours, the heat is turned off and the reaction is stirred at room temperature overnight.

The solution is refluxed for another 4 hours with removal of the distillate. The reaction is cooled and evaporated *in vacuo.* The residue is taken up in water and washed twice with ethyl acetate. A solid precipitate is formed in the ethyl acetate wash and collected by vacuum filtration. The solid is dissolved in is M NaOH, and acidified with concentrated HCl. The resulting solid is collected by vacuum filtration. After drying in *vacuo,* a white solid is obtained (m.p. 194-196°C).

The original aqueous layer is acidified with concentrated HCl and the solid is collected by vacuum filtration. This solid is recrystalized from ethyl acetate to afford light tan crystals (m.p. 195-196°C). The ¹H NMR and mass spectra of both solids are identical and consistent with the desired 2-[4-[2-(4-fluorophenyl)]pyrimidinyl]-3-pyridinecarboxylic acid.

### EXAMPLE 4

### Preparation of 2[4-[2-(3-chloro-4-methylphenyl)]pyrimidinyl]-3-pyridinecarboxylic acid (Compound 24 in Table 1).

To a solution of 3.47 g (14.8 mmol) 1-(3-methoxycarbonylpyridin-2-yl)-3-(N,N-dimethylamino)prop-2-en-1-one and 2.50 g (14.8 mmol) 3-chloro-4-methylbenzamidine in 150 mL methanol, is added 6.8 mL 25% sodium methoxide in methanol. The resulting solution is refluxed for 2 hours allowing about half of the methanol to distill off and stirred at ambient temperature over a weekend. Refluxing is continued for 4 additional hours, the mixture is cooled and evaporated *in vacuo.* The residue is treated with 1 M aqueous sodium hydroxide and filtered to remove an insoluble solid. The filtrate is washed with ethyl acetate, acidified to pH 3 with concentrated HCI, and extracted with 3 portions of ethyl acetate, which upon evaporation *in vacuo,* afforded a tan solid. The insoluble solid obtained from the initial filtration is suspended in 1 M aqueous sodium hydroxide at 50°C until dissolution, washed with ethyl acetate, filtered, and acidified with concentrated HCl. The resulting aqueous solution is extracted with 2 portions of ethyl acetate. The ethyl acetate extracts are washed with water, dried over magnesium sulfate, filtered, and evaporated *in vacuo* to give a light tan solid, which is combined with the 0.20 g of tan solid obtained above. The ¹H NMR and mass spectra of this combination (mp 168-172°C) are consistent with the desired 2-[4-[2-(3-chloro-4-methylphenyl)]pyrimidinyl]-3-pyridinecarboxylic acid.

### EXAMPLE 5

### Preparation of 2-[4-[2-(3-chlorophenoxy)methyl]pyrimidinyl]-3-pyridinecarboxylic acid (Compound 22 in Table 1).

To a solution of 2.66 g (11.4 mmol) 1-(3-methoxycarbonylpyridin-2-yl)-3-(N,N-dimethylamino)prop-2-en-1-one and 2.10 g (11.4 mmol) 3-chlorophenoxyacetamidine in 100 mL methanol, is added 5.2 mL (22.8 mmol) 25% sodium methoxide in methanol. The resulting solution is gently refluxed ovemight allowing about half of the methanol to distill off. The reaction mixture is evaporated *in vacuo* and the residue is partitioned between 0.5 M aqueous sodium hydroxide and ethyl acetate. The aqueous layer is washed with ethyl acetate, then acidified to pH 3 with concentrated HCI. The crude solid product is collected by vacuum filtration and purified by silica gel chromatography (2:1 ethyl acetate: methanol) to afford a tan solid (mp 155-158°C). The ¹H NMR and mass spectra of this solid are as expected for 2-[4-[2-(3-chlorophenoxy)methyl]pyrimidinyl]-3-pyridinecarboxylic acid.

### EXAMPLE 6

### Preparation of 2-[4-[2-(2-Phenyl)ethenyl]pyrimidine]-3-pyridinecarboxylic acid (Compound 26 in Table 1).

To a solution of 2.72 g (11.6 mmol) 1-(3-methoxycarbonylpyridin-2-yl)-3-(N,N-dimethylamino)prop-2-en-1-one and 1.86 g (11.6 mmol) cinnamamidine in 50 mL methanol, is added 8.0 mL 25% sodium methoxide in methanol. The resulting dark solution is refluxed for 2 hours with removal of the distillate. After stirring at ambient temperature for 72 hours, the reaction mixture is treated with 2.0 mL acetic acid and evaporated to dryness *in vacuo.* The residue is partitioned between saturated aqueous sodium bicarbonate and ethyl acetate. The aqueous layer is washed with two portions of ethyl acetate, acidified to pH 4 with concentrated HCl and filtered to remove crude solid product. The filtrate is extracted with ethyl acetate and evaporated *in vacuo* to yield a brown oil. The crude solid is combined with the brown oil and purified by thin layer preparatory chromatography on silica gel with 180:20:1 dichloromethane: methanol:acetic acid to afford a tan solid (mp 167°C decomposition). The ¹H NMR spectrum of this solid is as expected for 2-[4-[2-(2-phenyl)ethenyl]pyrimidinyl]-3-pyridinecarboxylic acid.

### EXAMPLE 7

### Preparation of 2-[4-[2-(1-naphthyl)]pyrimidinyl]-3-pyridinecarboxylic acid (Compound 27 in Table 1).

To a solution of 4.46 g (19 mmol) 1-(3-methoxycarbonylpyridin-2-yl)-3-(N,N-dimethylamino)prop-2-en-1-one and 3.24 g (19 mmol) 1-naphthamidine in 50 mL methanol, is added 13 mL (57 mmol) 25% sodium methoxide in methanol. The resulting dark solution is refluxed for 18 hours. The reaction mixture is treated with 3.3 mL acetic acid and evaporated *in vacuo.* The residue is chromatographed on silica gel with 4:1 ethyl acetate:methanol. The product containing fractions are evaporated *in vacuo,* suspended in ethyl acetate, filtered and evaporated *in vacuo* to yield slightly impure product. This solid is rechromatographed on silica gel with 90:10:1 ethyl acetate:methanol:acetic acid to finally afford a tan solid (mp 205-207°C). The ¹H NMR spectrum of this solid is as expected for 2-[4-[2-(1-naphthyl)]-pyrimidinyl]-3pyridinecarboxylic acid.

These and other compounds which can be made by the foregoing processes are set forth in Tables 1 and 2 which follow, wherein the various substituent groups are indicated.

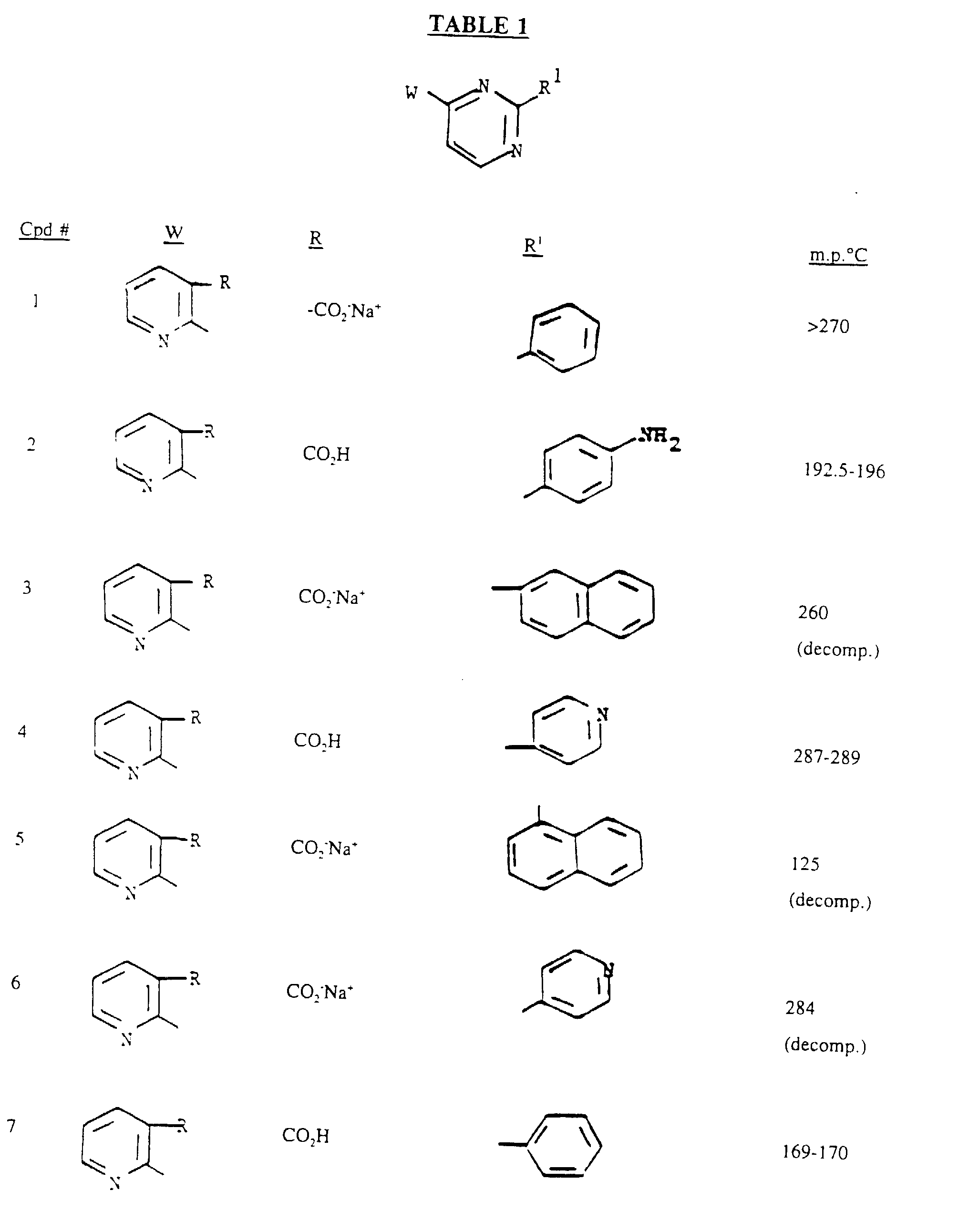

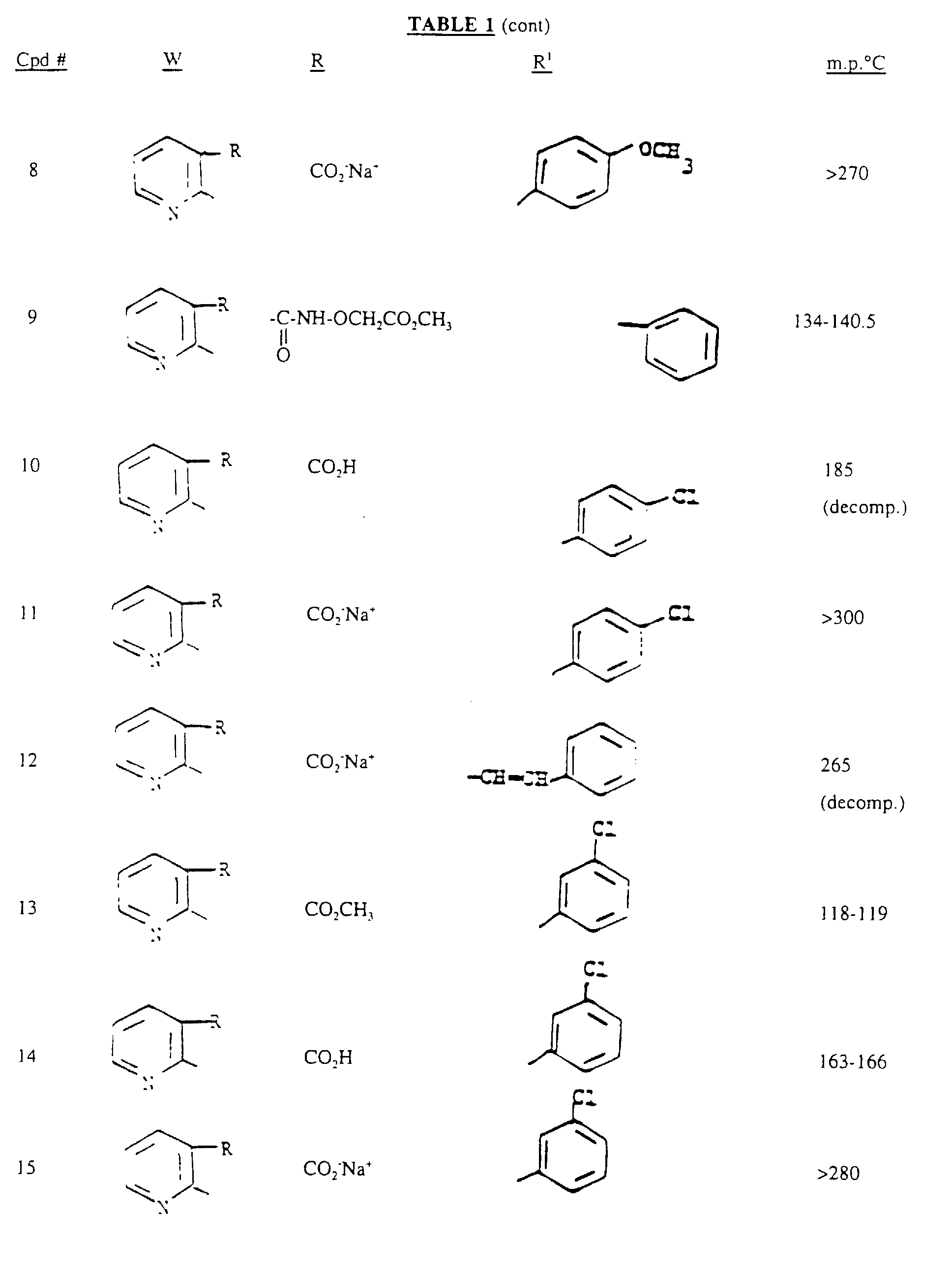

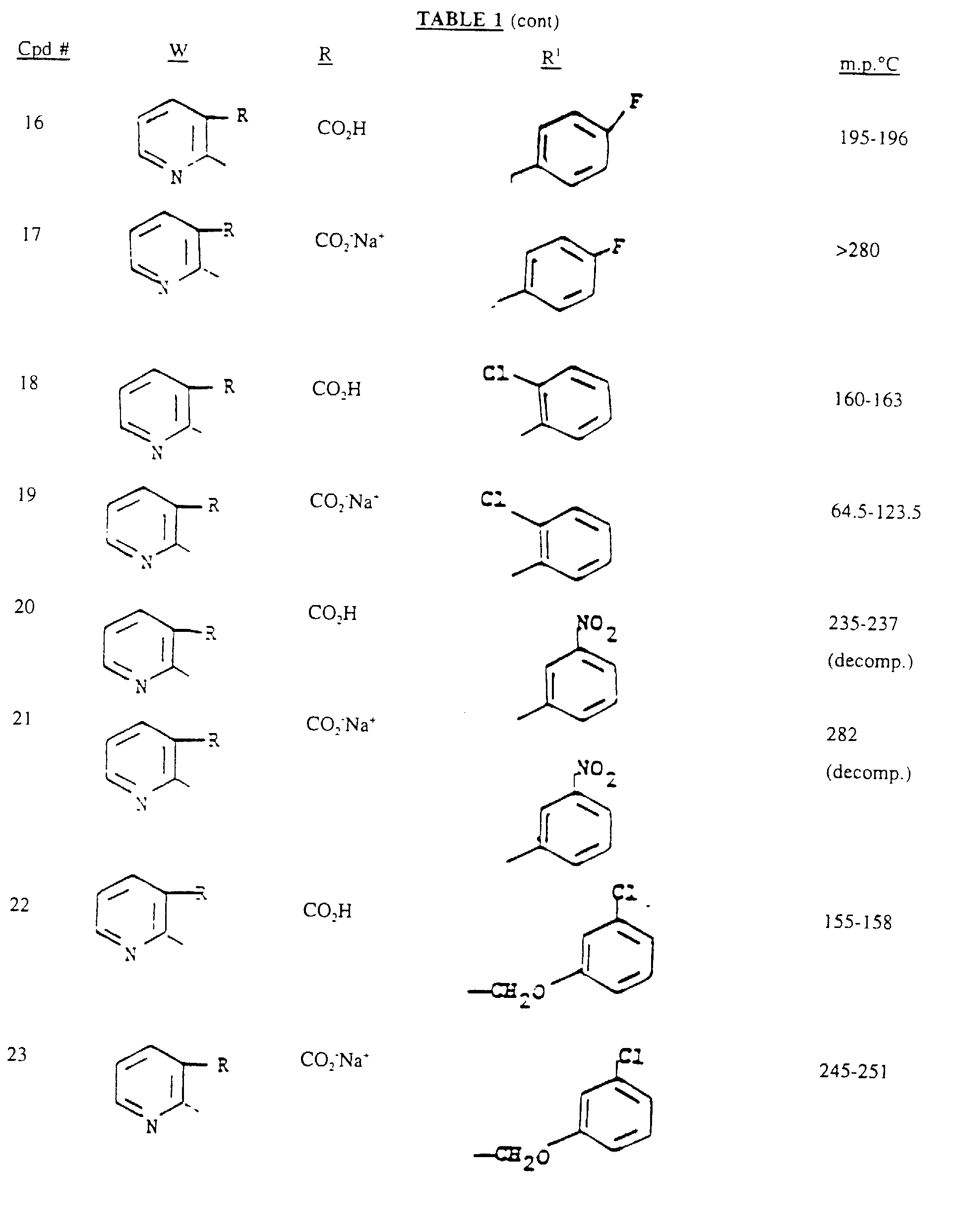

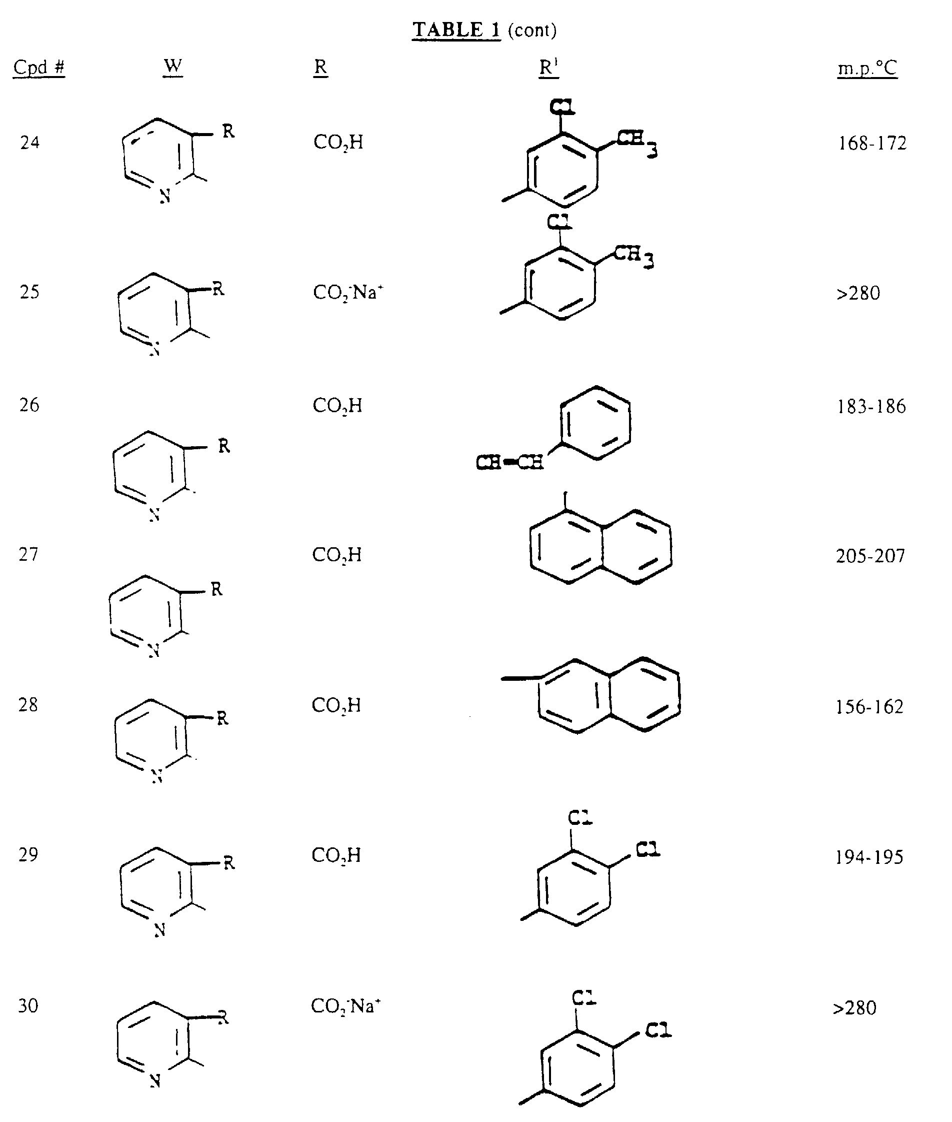

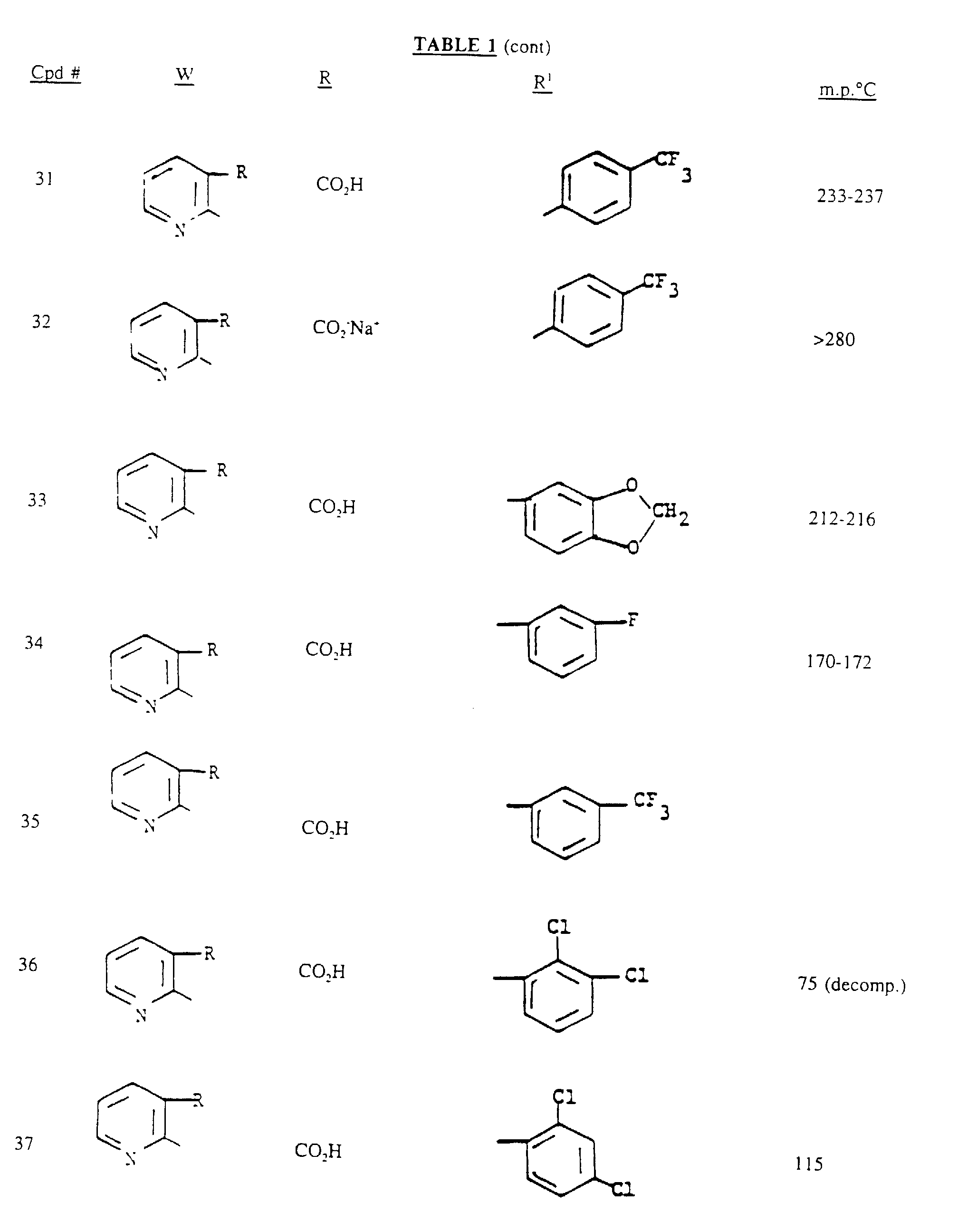

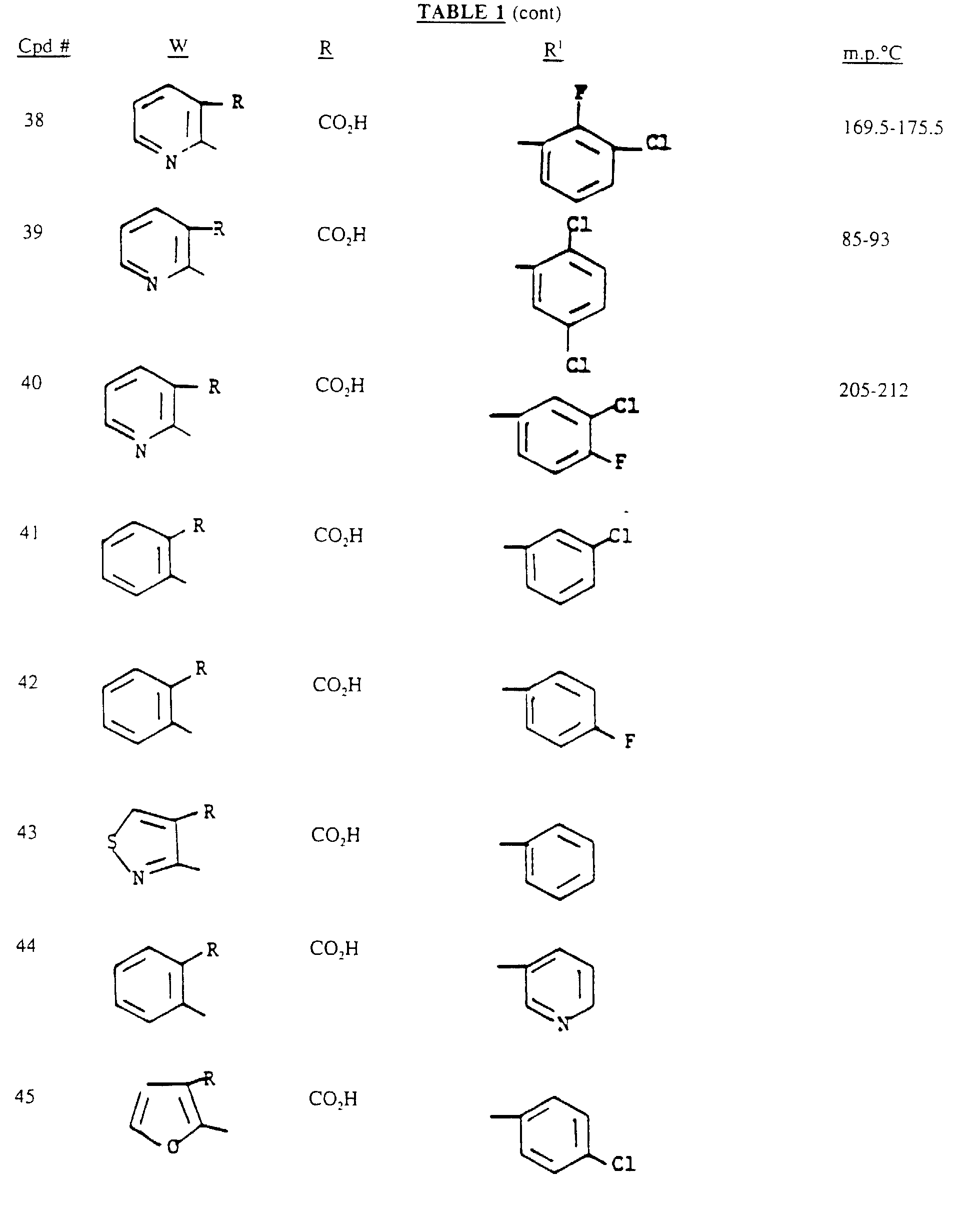

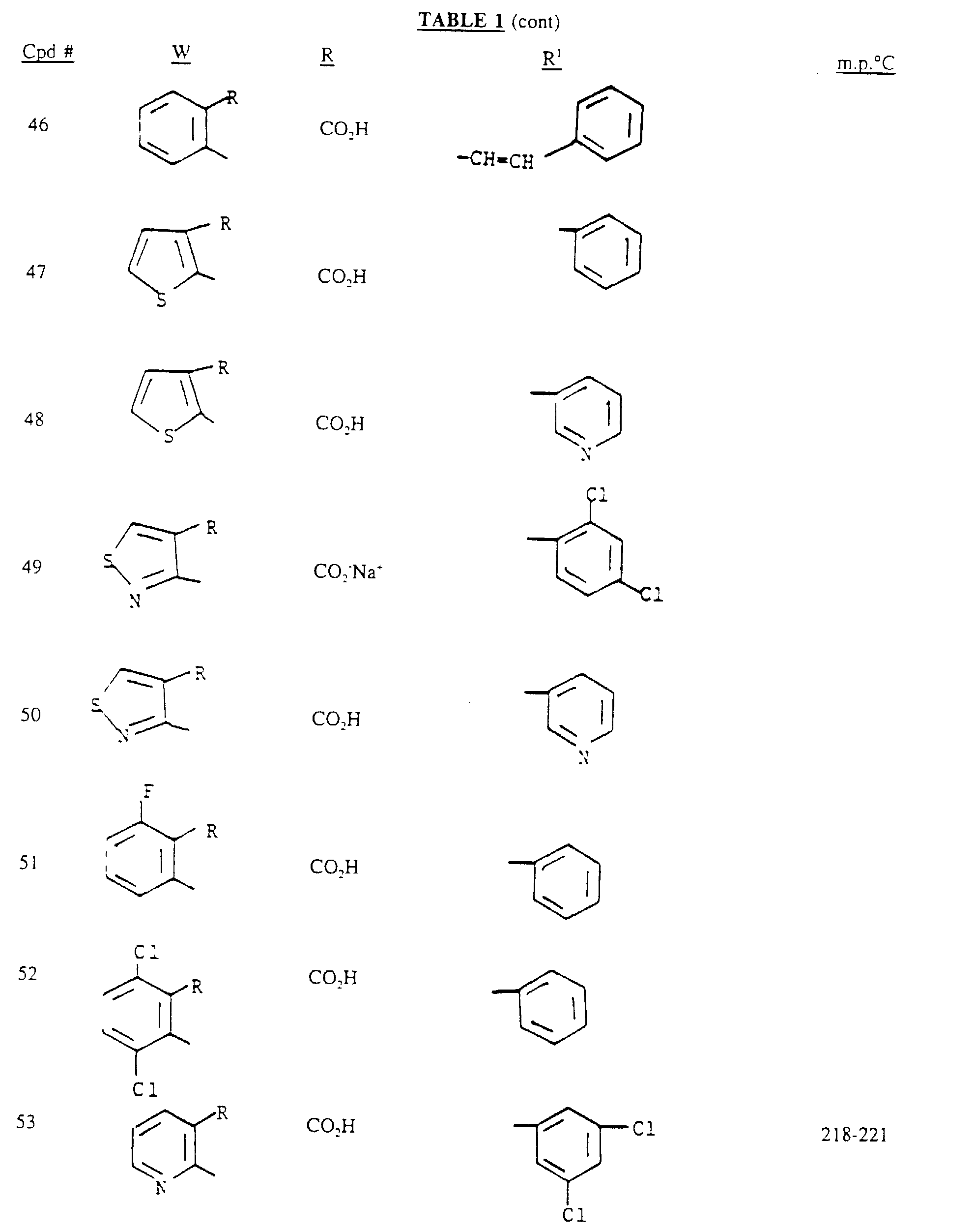

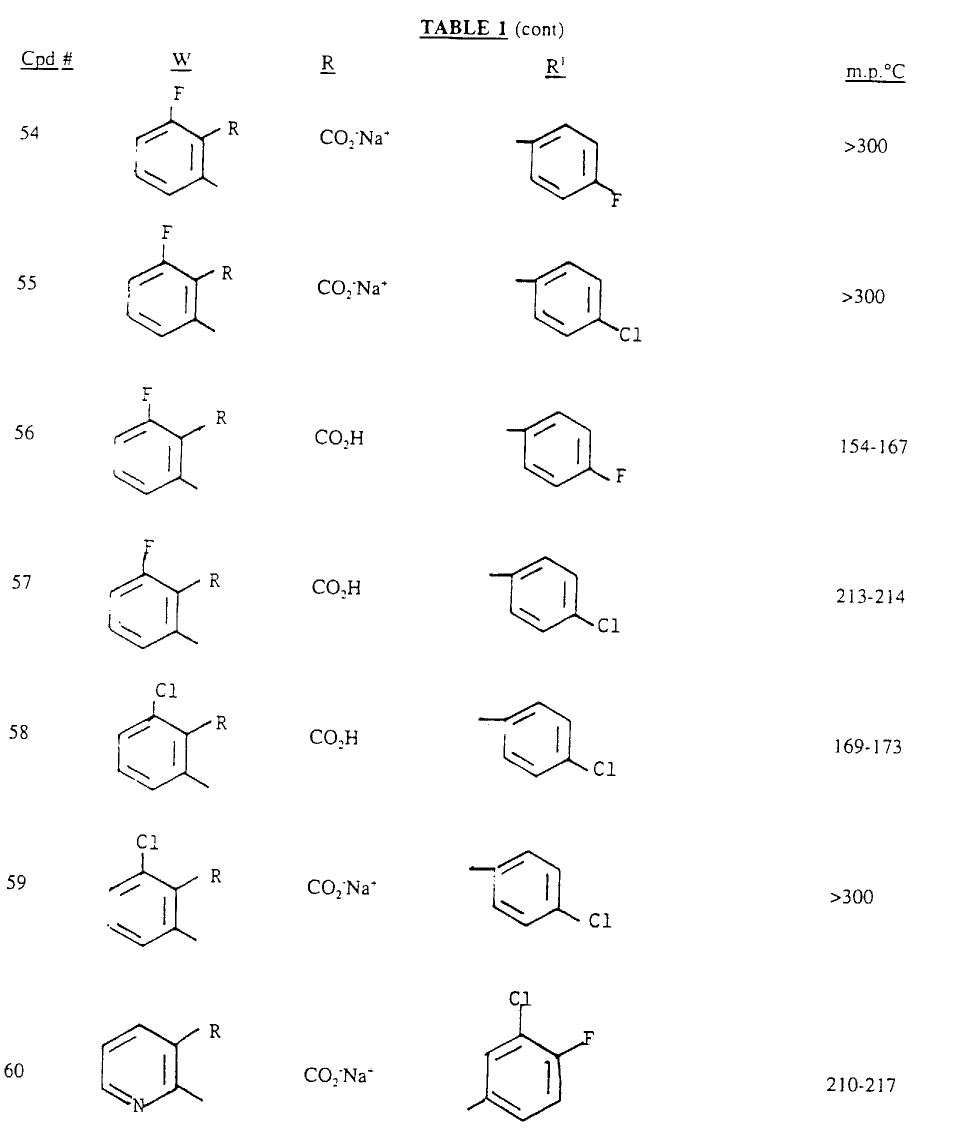

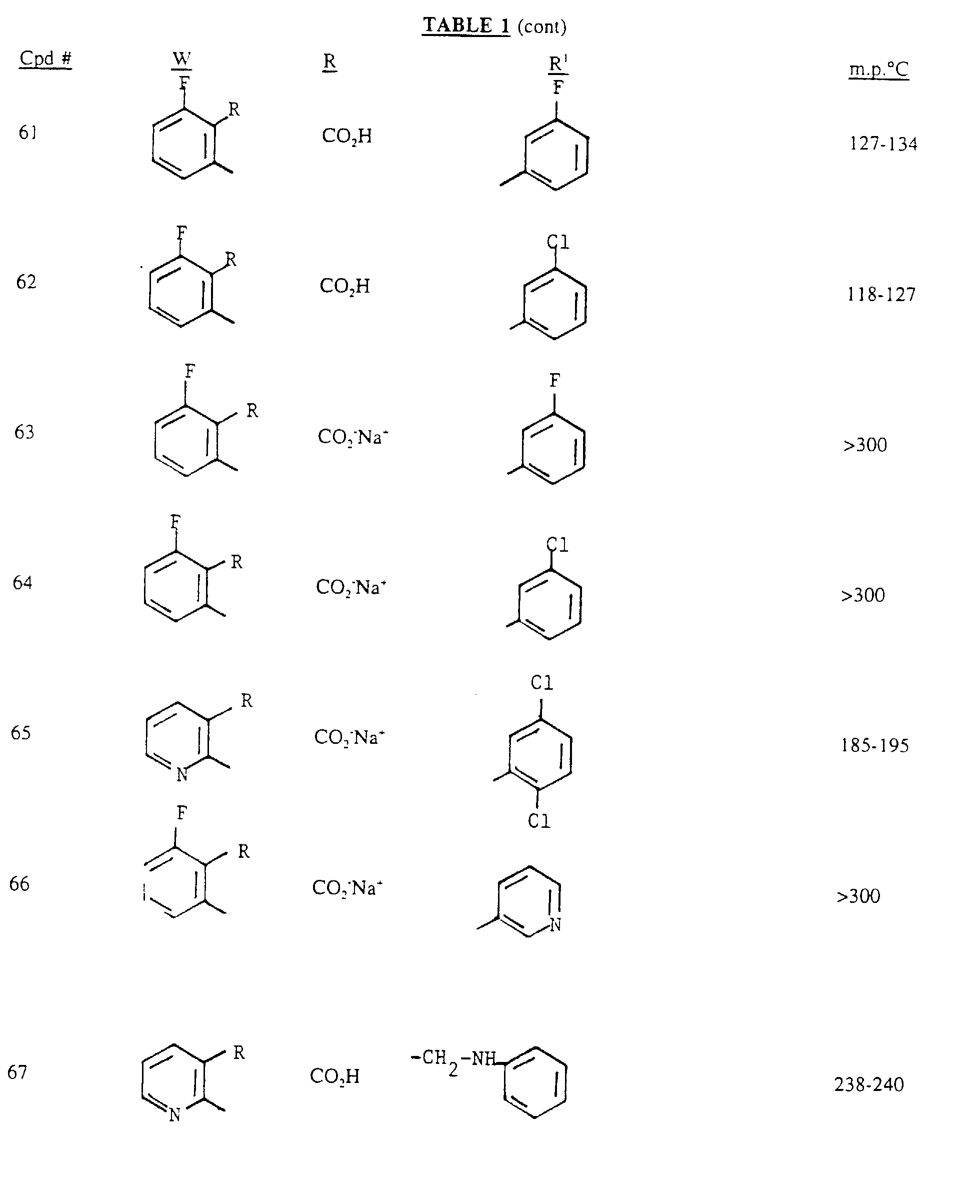

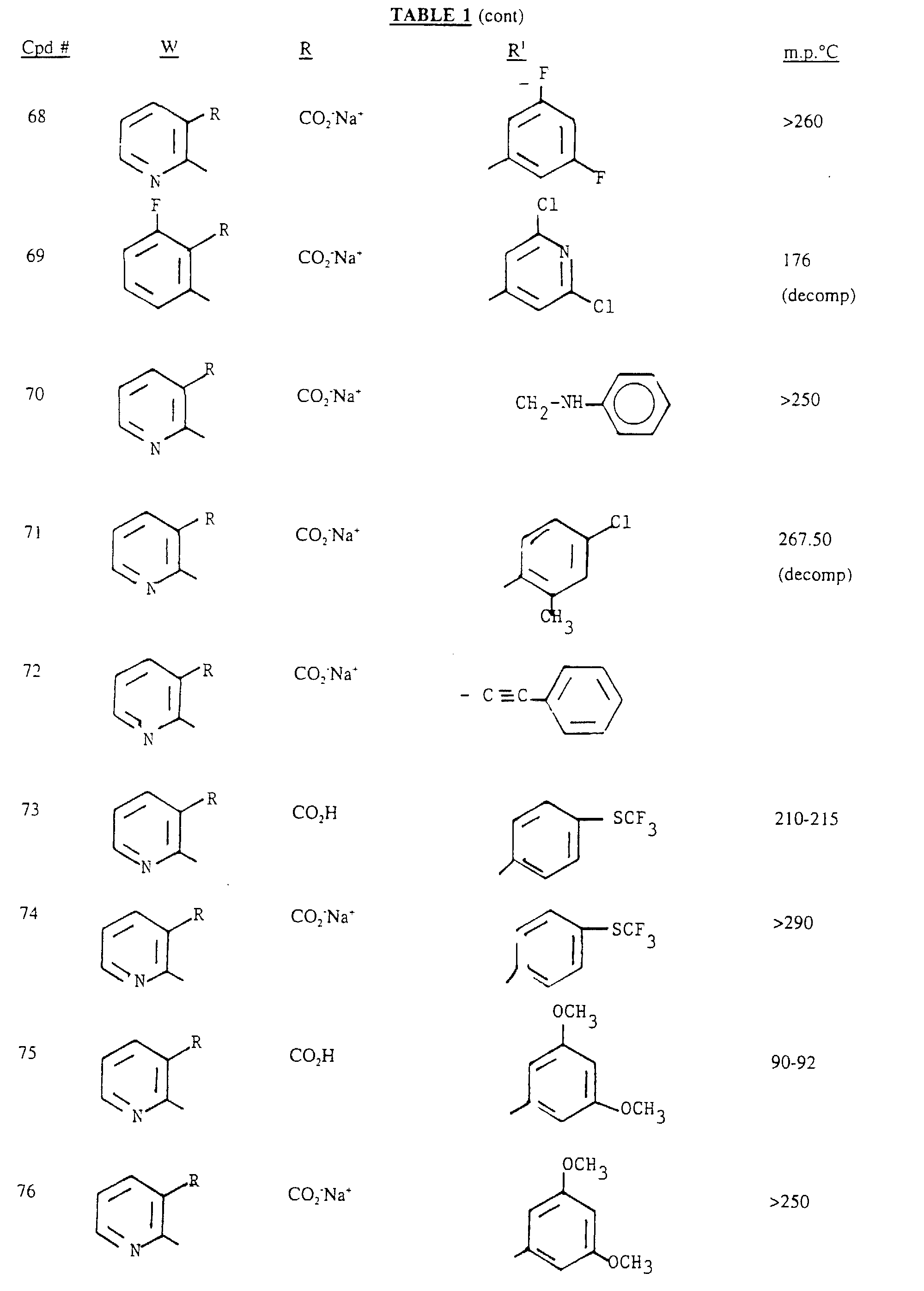

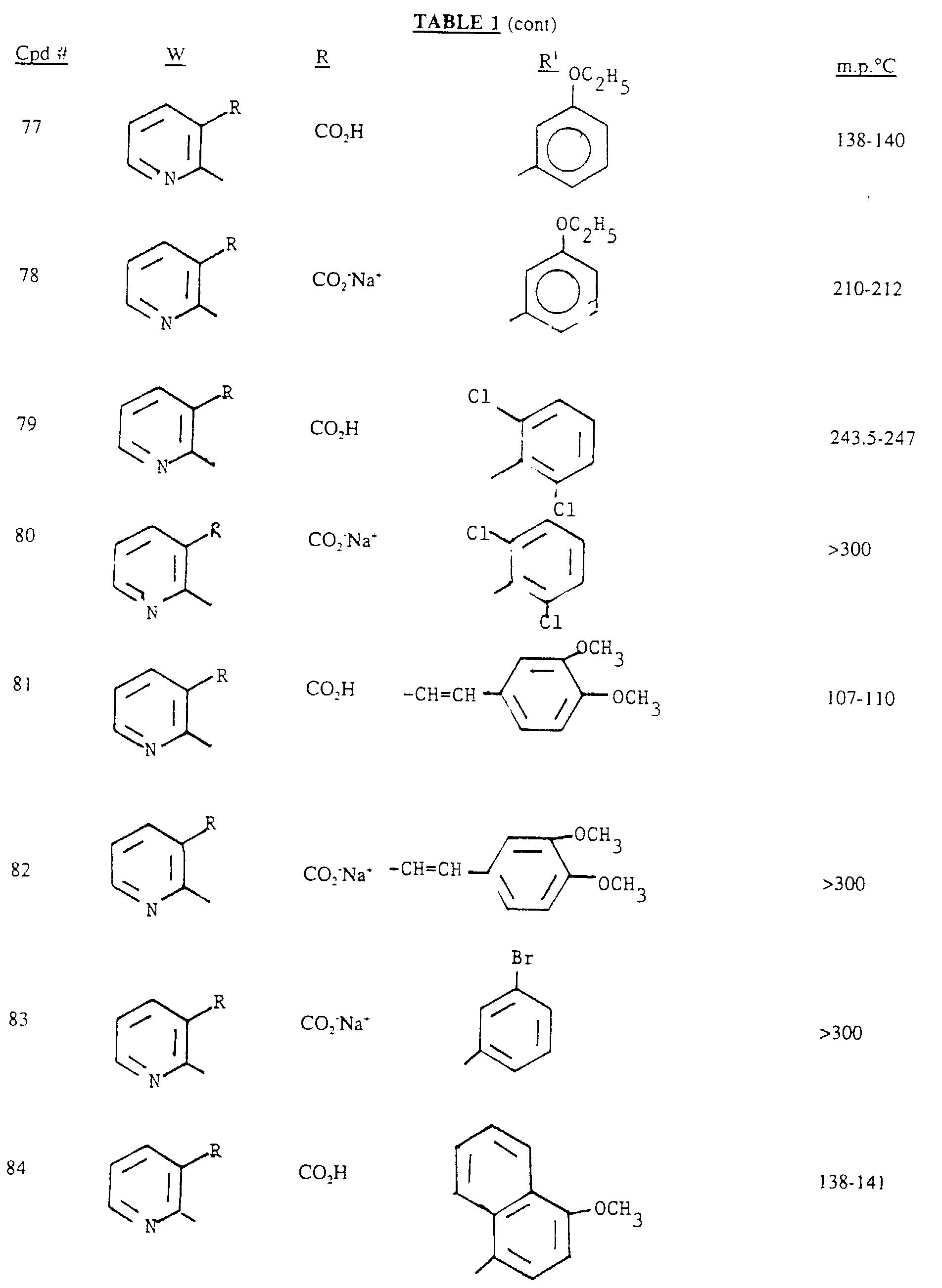

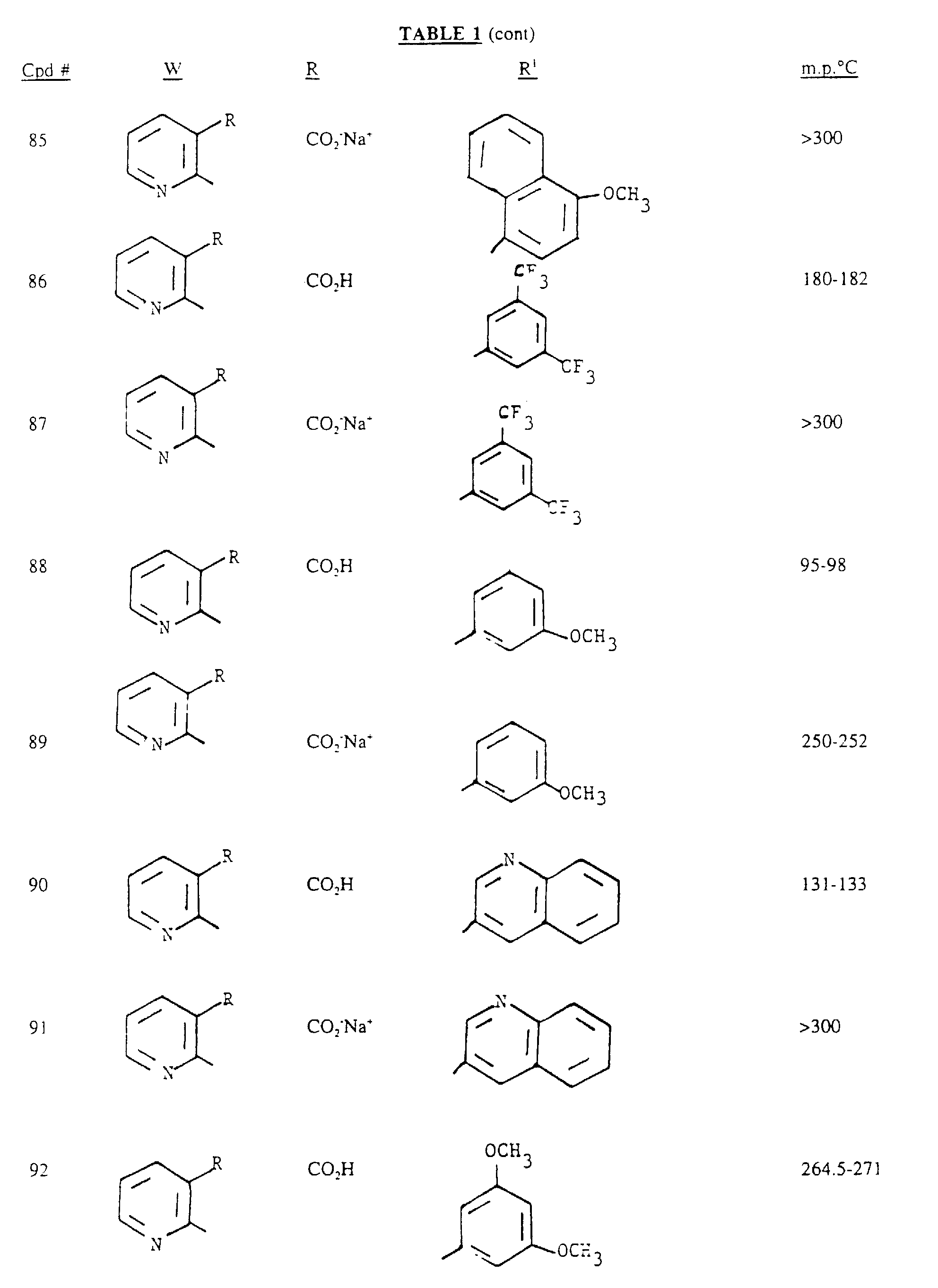

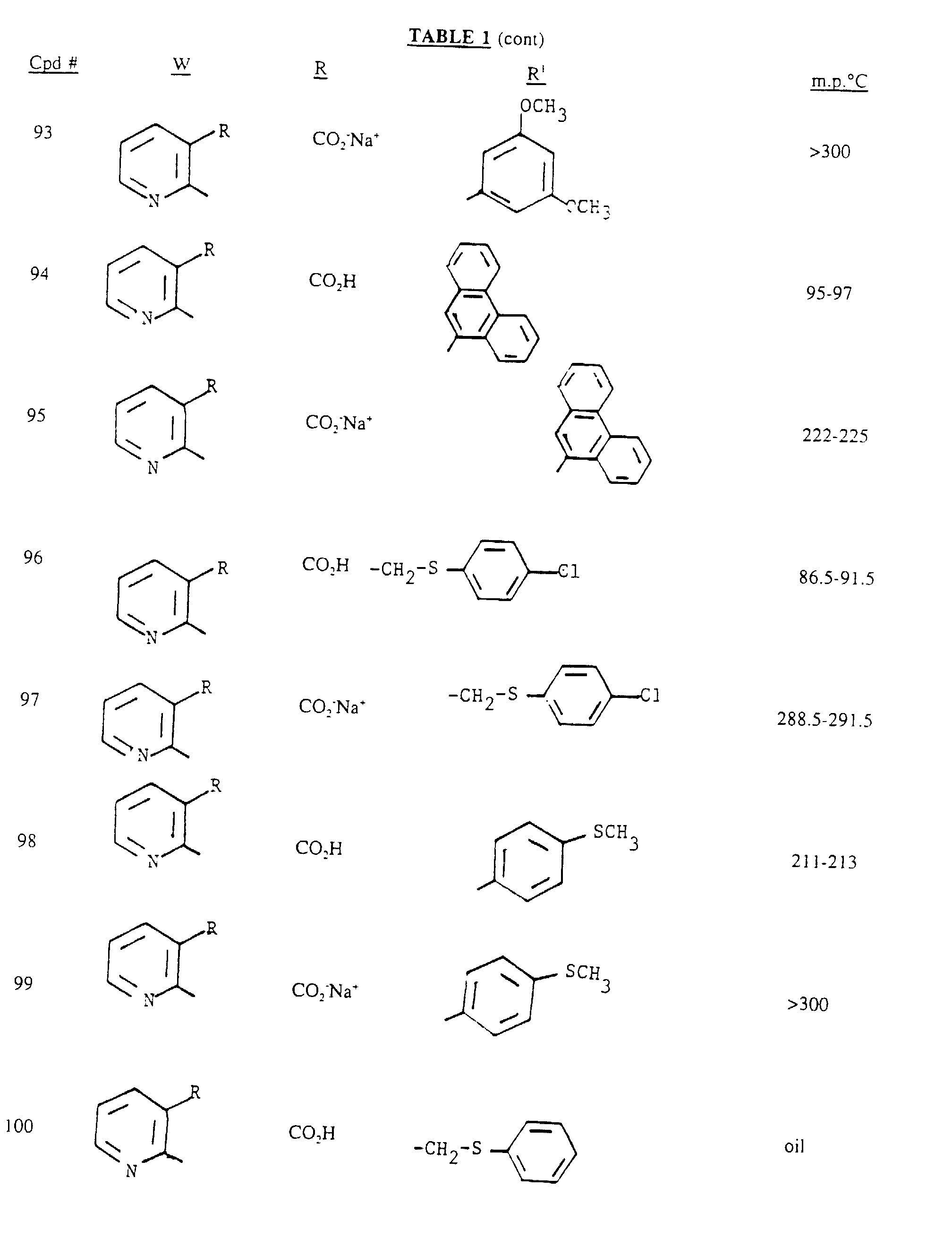

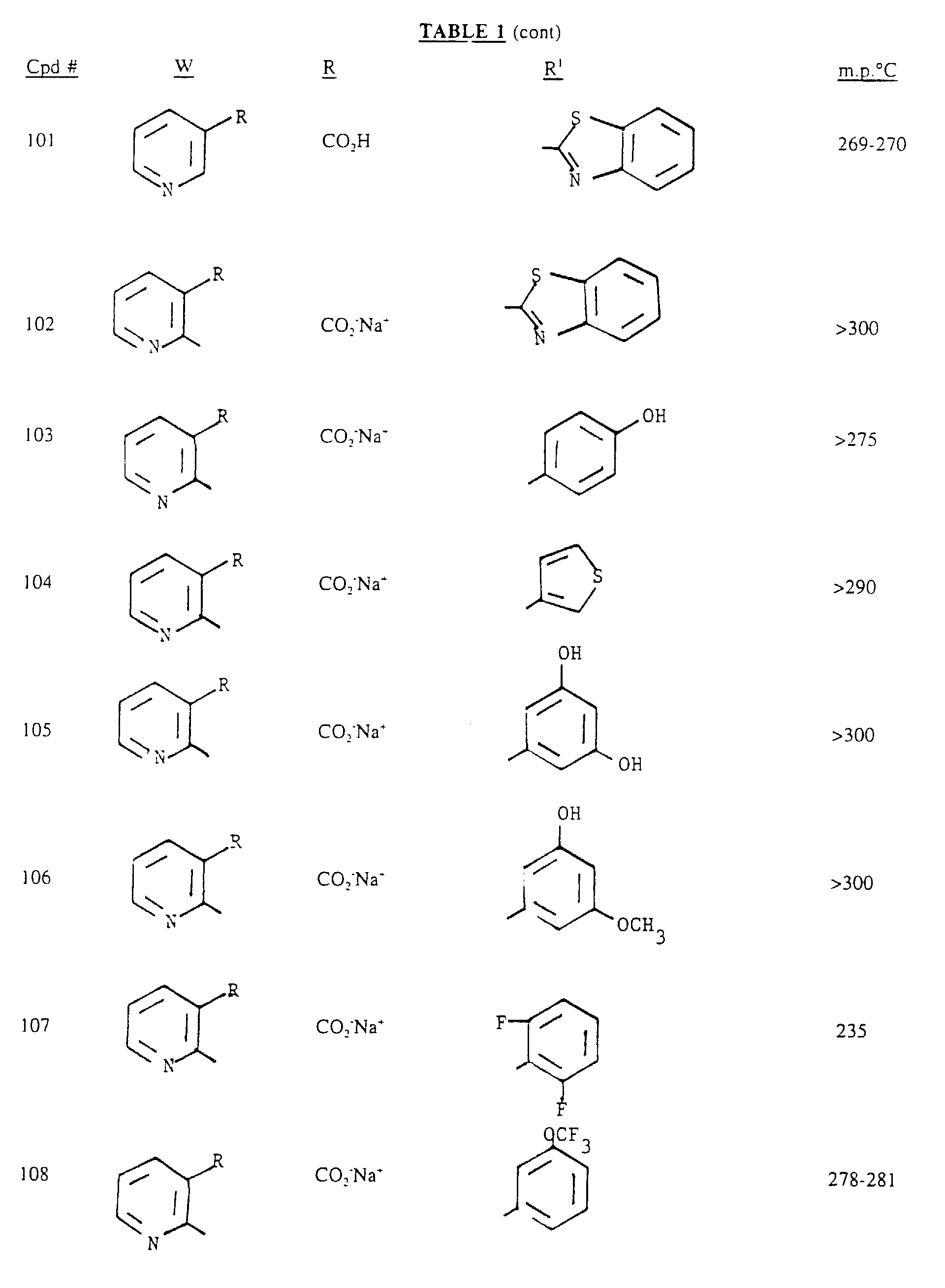

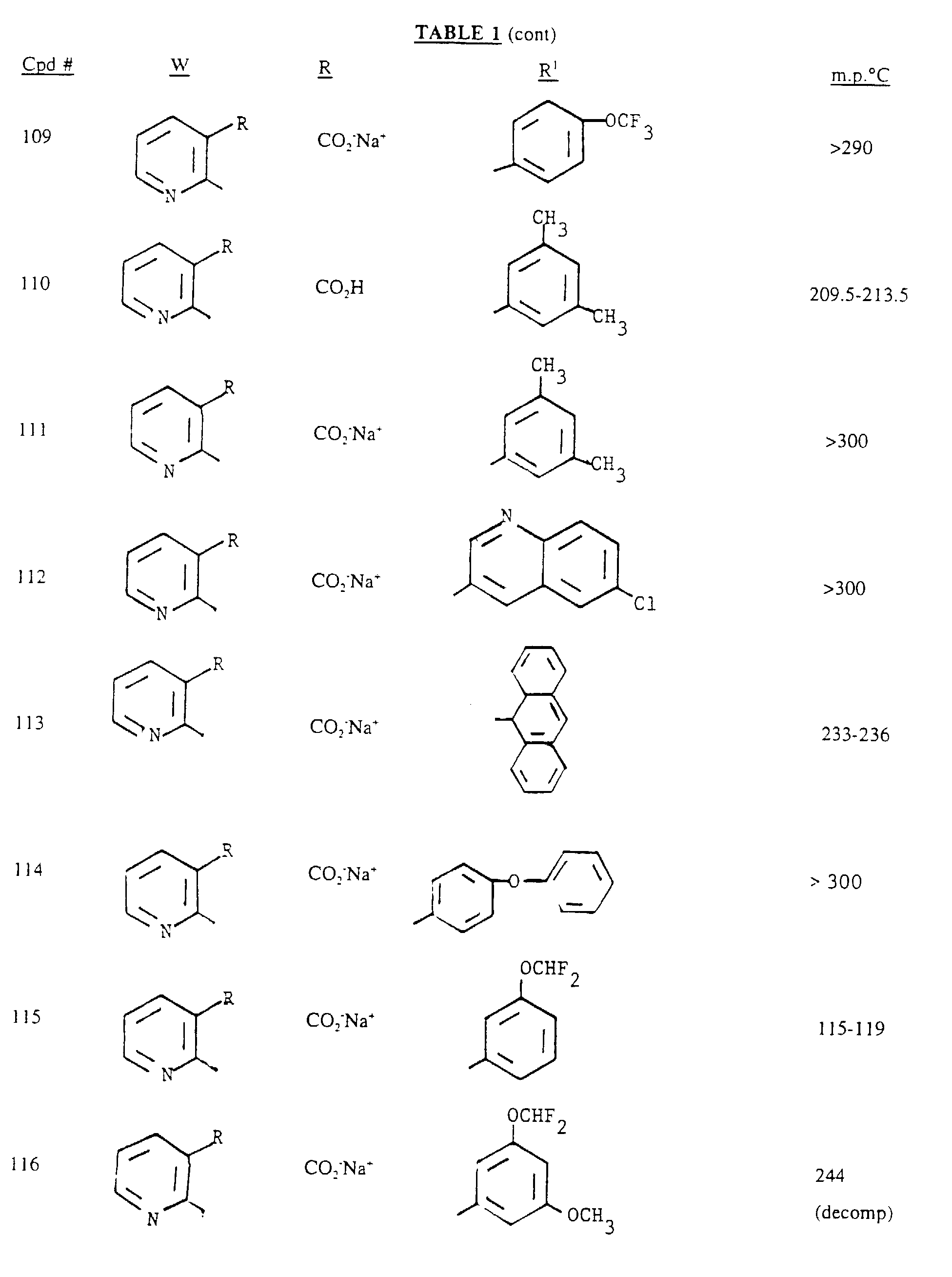

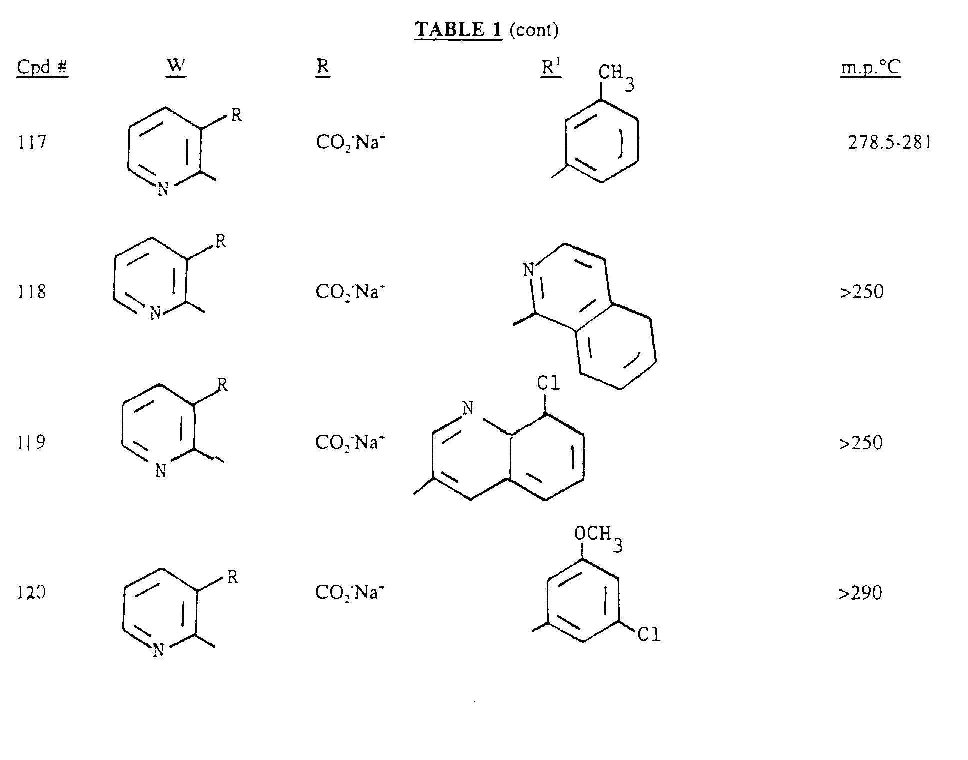

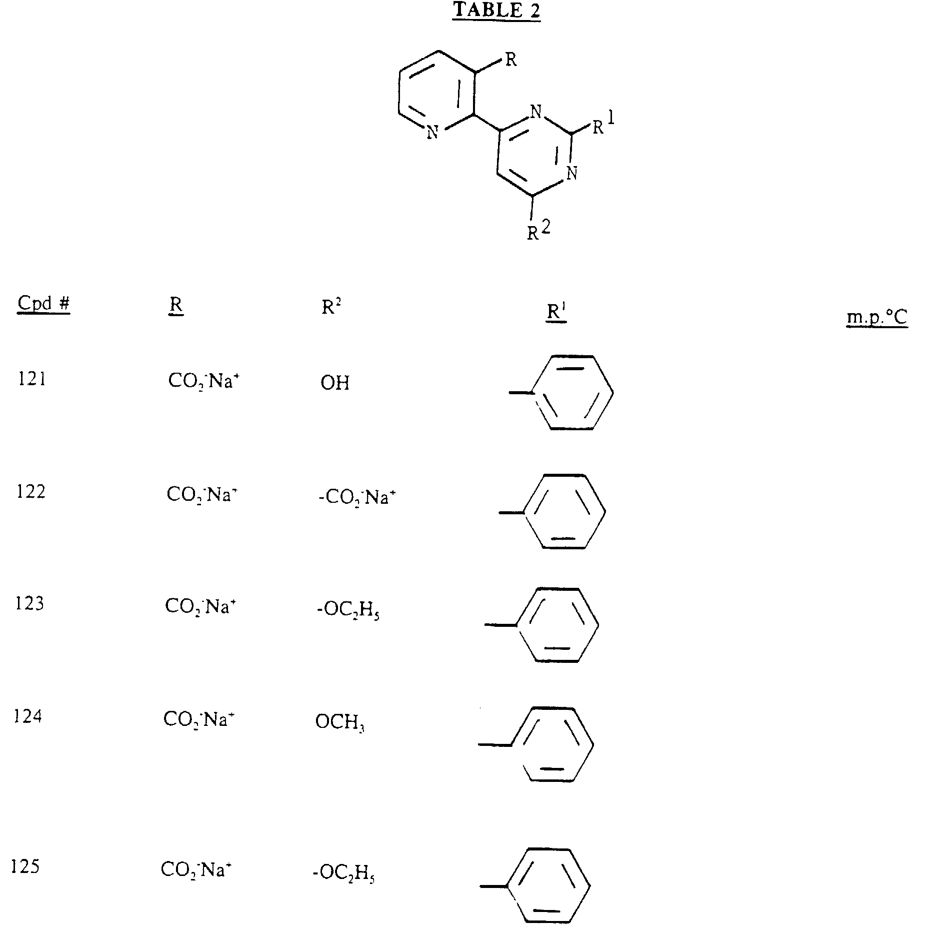

### EXAMPLE 8

The test compounds are weighed and dissolved in a stock solution consisting of acetone:deionized water (1:1) and 0.5% adjuvant mixture. Dilutions from this stock solution are performed to allow for preparation of spray solutions consisting of single doses applied at a level equivalent to either 4.0, 1.0 or 0.25 kg/ha of active ingredient. The solutions are applied by a linear track sprayer set to deliver 1000 L/ha spray volume.

In pre-emergent studies, each dose of herbicide is applied as a band treatment over the seed zone. Pots containing the seeds are then top-dressed with soil, the plants are grown in the greenhouse and visually evaluated 7 and 19 days after treatment.

In post-emergence studies, each dose of compound is applied to the foliage of the selected weed seedling species. The plants are allowed to grow in the greenhouse and visually evaluated at 1, 7 and 19 days after treatment. Weed species tested are shown in Table 3. Some compounds of formula I showed activity in the pre-emergent and post emergent studies. Herbicidal control is evaluated as % injury with 100% injury considered complete control. At an application rate of 1.0 kg/ha active ingredient the compounds 1, 3, 5, 7-12, and 19-24 exhibited herbicidal control at greater than 80% for various tested weeds in both pre-emergence and post-emergence screenings.

In pre-emergence screening on grasses the compounds 60-65, 68, 71-78, 83, 84 and 94 provided greater than 75% control at 1.0 kg/ha on all tested weed species. It is understood that this list does not reflect all obtained data nor encompass all compounds which achieved the given limit.

**TABLE 3**

| Common Name | Genus Species |
|---|---|
| Velvetleaf | Abutilon theophrasti |
| Redroot Pigweed | Amaranthus retroflexus |
| Mustard White | Sinapis alba |
| Black Nightshade | Solanum nigrum |
| Wild Oat | Avena fatua |
| Downy Brome | Bromus tectorum |
| Barnyardgrass | Echinochloa crus-gali |
| Green Foxtail | Setaria viridis |

### METHODS OF APPLICATION

Application of a compound of formula I is made according to conventional procedure to the weeds or their locus using a herbicidally effective amount of the compound, usually from 1 g to 10 kg/ha.

Compounds according to the invention may be used for the control of both broadleaf and grassy weeds in both preplant incorporation and pre- and post-emergent application. Compounds may also exhibit selectivity in various crops and may thus be suited for use in weed control in crops such as but not limited to corn, cotton, wheat, soybean and rice.

The optimum usage of a compound of formula I is readily determined by one of ordinary skill in the art using routine testing such as greenhouse testing and small plot field testing. It will depend on the compound employed, the desired effect (a phytotoxic effect requiring a higher rate than a plant growth regulating effect), the conditions of treatment and the like. In general, satisfactory phytotoxic effects are obtained when the compound of formula I is applied at a rate in the range of from 0.001 to 5.0 kg, more preferably of from 0.05 to 2.5 kg per hectare, especially 0.01 to 2.5 kg per hectare.

The compounds of formula I may be advantageously combined with other herbicides for broad spectrum weed control. Examples of herbicides which can be combined with a compound of the present invention include those selected from carbamates, thiocarbamates, chloroacetamides, triazines, dinitroanilines, benzoic acids, glycerol ethers, pyridazinones, uracils, phenoxys and ureas for controling a broad spectrum of weeds.

The compounds of formula I are conveniently employed as herbicidal compositions in association with agriculturally acceptable diluents. Such compositions also form part of the present invention. They may contain, aside from a compound of formula I as active agent, other active agents, such as herbicides or compounds having antidotal, fungicidal, insecticidal or insect attractant activity. They may be employed in either solid or liquid forms such as a wettable powder, an emulsifiable concentrate, a granule or a microcapsule incorporating conventional diluents. Such compositions may be produced in conventional manner, for example by mixing the active ingredient with a diluent and optionally other formulating ingredients such as surfactants.

Agriculturally acceptable additives may be employed in herbicidal compositions to improve the performance of the active ingredient and to reduce foaming, caking and corrosion, for example.

The term "diluent" as used herein means any liquid or solid agriculturally acceptable material which may be added to the active constituent to bring it in an easier or improved applicable form, respectively, to a usable or desirable strength of activity. It can for example be talc, kaolin, diatomaceous earth, xylene or water.

"Surfactant" as used herein means an agriculturally acceptable material which imparts emulsifiability, spreading, wetting, dispersibility or other surface-modifying properties. Examples of surfactants are sodium lignin sulfonate and lauryl sulfate.

Particularly formulations to be applied in spraying forms such as water dispersible concentrates or wettable powders may contain surfactants such as wetting and dispersing agents, for example the condensation product of formaldehyde with naphthylene sulphonate, an ethoxylated alkylphenol and an ethoxylated fatty alcohol.

In general, the formulations include from 0.01 to 99% by weight of active agent and from 0 to 20% by weight of agriculturally acceptable surfactant, and from 0.1 to 99.99% of solid or liquid diluent(s) the active agent consisting either of at least one compound of formula I or mixtures thereof with other active agents. Concentrate forms of compositions generally contain between about 2 and 95%, preferably between about 10 and 90% by weight of active agent.

Typical herbicidal compositions, according to this invention, are illustrated by the following Examples A, B, C, D and E in which the quantities are in parts by weight.

### EXAMPLE A

### Preparation of a Soluble Powder

The water soluble salts of this invention can be hammer milled to a screen size of 100 mesh. The resulting powder will readily dissolve in water for spraying.

### EXAMPLE B

### Preparation of a Wettable Powder

25 Parts of a compound according to this invention are mixed and milled with 25 parts of synthetic fine silica, 2 parts of sodium lauryl sulphate, 3 parts of sodium lignosulfonate and 45 parts of finely divided kaolin until the mean particle size is about 5 micron. The resulting wettable powder is diluted with water to a desired concentration.

### EXAMPLE C

### Preparation of Water Dispersible Granule

40 Parts of a water insoluble parent acid compound according to this invention are wet milled in a solution of 10 parts MARASPERSE N-22 (a sodium lignosulfonate) and 50 parts water until a median particle size of 5 micron is reached. The slurry is spray dried on a NIRRO MOBILE MINOR unit at an inlet temperature of 150°C and outlet temperature of 70°C. The resulting granule can be readily dispersed in water for application.

### EXAMPLE D

Preparation of a Microcapsule Suspension
(a) 0.38 Parts of a VINOL 205 (a partially hydrolyzed polyvinyl alcohol) are dissolved in 79.34 parts water.
(b) 3.75 Parts of an organic soluble parent acid compound according to this invention are dissolved in 3.75 parts TENNECO 500-100 (a xylene range aromatic solvent). To this solution are added 0.63 parts of SEBACOYL CHLORIDE and 0.88 parts PAPI 135 (polymethylene isocyanate).
(c) 1.89 Parts piperazine and 0.50 parts of NaOH are dissolved in 12.60 parts of water.

Transfer premix (a) to a one quart osterizer and while stirring add premix (b) and sheer for approximately 60 seconds or until a droplet size of 10-20 microns is reached. Immediately add premix (c), continue stirring for 3 hours and neutralize with acetic acid. The resulting capsule suspension may be diluted in water for spraying.

### EXAMPLE E

### Preparation of an Emulsifiable Concentrate

13 Parts of an organic soluble parent acid compound according to this invention are dissolved in 79 parts of TENNECO 500-100 along with 2 parts TOXIMUL RHF and 6 parts TOXIMUL S. TOXIMULS are a "matched pair"; each containing anionic and nonionic emulsifiers. The stable solution will spontaneously emulsify in water for spraying.

## Claims

1. A compound of the formula 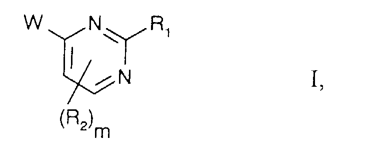 wherein
W is 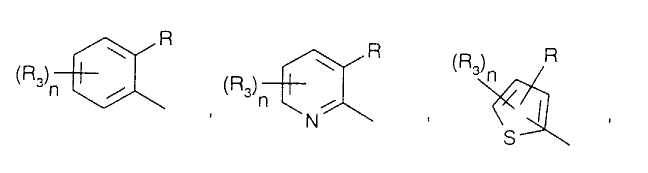 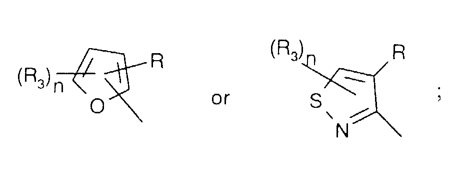 R³ is independently hydrogen, halogen, C₁-C₆-alkyl, C₁-C₆-haloalkyl, C₁-C₆-alkoxy, cyano, C₁-C₆-alkoxycarbonyl, C₁-C₆-alkylamino, di-C₁-C₆-alkylamino, or -N(R⁵)-CO-R⁶;
n is 1 to 4;
R is CO₂R⁴, CHO, CONH-O-CH₂CO₂R⁴, COSR⁴, CO₂CHR⁵OCOR⁶ or CH=NOR⁴;
R¹ is Ar, (Z)ₓY-Ar, or ZAr wherein Ar is an optionally substituted aryl or hetero aryl group selected from the group consisting of phenyl, pyridyl, piperonyl, naphthyl, indolyl, quinolyl, isoquinolyl, quinoxalinyl, quinazolinyl, benzoxazolyl, benzothiazolyl, phenanthryl, pyridyl-N-oxide, anthranilyl, pyrimidinyl, pyrazinyl, thienyl, furyl, pyrrolyl, oxazolyl, thiazolyl, isoxazolyl, isothiazolyl, imidazolyl, pyrazolyl, oxadiazolyl and thiodiazolyl; wherein the optional substituents are phenoxy, halo, C₁-C₆-alkyl, C₂-C₆-alkenyl, C₁-C₆-haloalkyl, C₁-C₆-haloalkylthio, C₁-C₆-alkylthio, C₁-C₆-alkylsulfinyl, C₁-C₆-alkylsulfonyl, C₁-C₆-alkoxy, C₁-C₆-haloalkoxy, C₁-C₆-alkoxy-C₁-C₆-alkyl, cyano, nitro, C₁-C₆-alkoxycarbonyl, amino, C₁-C₆-alkylamino, di-C₁-C₆-alkylamino or hydroxy; Y is O, S or NH; Z is optionally substituted C₁-C₃alkyl, C₂-C₄alkynyl, or an optionally substituted C₂-C₄alkenyl, wherein the substituents are independently C₁-C₆-alkyl and halogen;
x is 0 to 2;
R² is independently hydrogen, halogen, C₁-C₆-alkyl, C₂-C₆-alkenyl, C₁-C₆-haloalkyl, C₁-C₆-alkylthio, C₁-C₆-alkylsulfinyl, C₁-C₆-alkylsulfonyl C₁-C₆-alkoxy, C₁-C₆-alkoxy-C₁-C₆-alkyl, cyano, nitro, amino, C₁-C₆-alkylamino, di-C₁-C₆-alkylamino, CO₂R⁴ or hydroxy;
m is 1 to 2;
R⁴ is hydrogen, alkali or alkaline earth cation, ammonium cation, substituted ammonium cation, phosphonium cation, C₁-C₆-alkyl phosphonium cation, tri-C₁-C₆-alkylsulfonium cation, tri-C₁-C₆-alkylsulfoxonium cation, C₁-C₆-alkyl, C₂-C₆-alkenyl, C₁-C₆-haloalkyl, C₁-C₆-alkoxy-C₁-C₆-alkyl, optionally substituted phenyl or optionally substituted phenyl-C₁-C₆-alkyl;
R⁵ is hydrogen or C₁-C₆-alkyl; and
R⁶ is C₁-C₆-alkyl, C₂-C₆-alkenyl, C₁-C₆-haloalkyl, C₁-C₆-alkoxy,-C₁-C₆-alkyl, optionally substituted phenyl or optionally substituted phenyl-C₁-C₆-alkyl, wherein the optional substituents are C₁-C₆-alkyl, C₁-C₆-haloalkyl, halogen, C₁-C₆-alkoxy, C₂-C₆-alkenyl, cyano and nitro, and provided that (i) when R¹ is phenyl; W is not 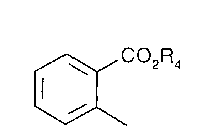 and (ii) when R¹ is optionally substituted phenyl and W is 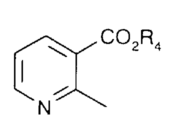
R² is not C₁-C₆-alkyl or C₂-C₆-alkenyl.

2. A compound according to Claim 1 wherein W is 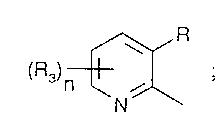
R is CO₂R⁴; CHO; CONH-O-CH₂CO₂R⁴; COSR⁴; CO₂CHR⁵OCOR⁶ or CH=NOR⁴;
R¹ is Ar, ZₓYAr or ZAr wherein Ar is an optionally substituted phenyl, pyridyl, naphthyl, indolyl, piperonyl, quinolyl, benzoxazolyl, benzothiazolyl, anthranilyl or phenanthryl;
R² is hydrogen, CO₂R⁴ or C₁-C₆-alkoxy; and
R³ is hydrogen or halogen.

3. A compound according to Claim 2 wherein R is CO₂CHR⁵OCOR⁶ or CO₂R⁴; R⁴ is hydrogen, Na, NH₄, K, Ca, Mg, trimethylsulfonium, trimethyl sulfoxonium or isopropylammonium, R¹ is optionally substituted phenyl, pyridyl, naphthyl, piperonyl, quinolyl, (Z)phenyl, or (Z)ₓOphenyl; Z is C₁-C₃alkyl, C₂-C₄alkynyl or C₂-C₄alkenyl and x is 1.

4. A compound according to Claim 1 wherein W is 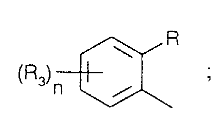
R is CO₂R⁴; CHO; CONH-O-CH₂CO₂R⁴; COSR⁴; CO₂CHR⁵OCOR⁶ or CH=NOR⁴;
R¹ is Ar, ZₓYAr or ZAr wherein Ar is an optionally substituted phenyl, pyridyl, naphthyl, indolyl, piperonyl, quinolyl, benzoxazolyl, benzothiazolyl, anthranilyl or phenanthryl;
R² is hydrogen, C₁-C₆-alkyl, CO₂R⁴, or C₁-C₆-alkoxy; and
R³ is hydrogen or halogen.

5. A compound according to Claim 2 wherein R is CO₂CHR⁵OCOR⁶ or CO₂R⁴; R⁴ is hydrogen, Na, NH₄, K, Ca, Mg, trimethylsulfonium, trimethyl sulfoxonium or isopropylammonium; R² is hydrogen and R¹ is optionally substituted phenyl, pyridyl, naphthyl, piperonyl, quinolyl, (Z)phenyl, or (Z)ₓOphenyl and x is 1.

6. A compound according to Claim 1 wherein W is 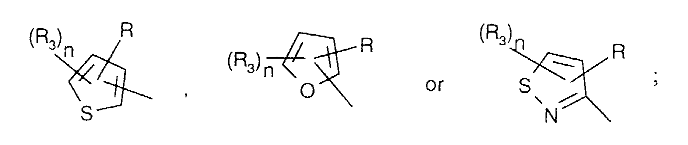
R is CO₂R⁴; CHO; CONH-O-CH₂CO₂R⁴; COSR⁴; CO₂CHR⁵OCOR⁶ or CH=NOR⁴;
R¹ is Ar, ZₓOAr or ZAr wherein Ar is an optionally substituted phenyl, pyridyl, naphthyl, indolyl, piperonyl, quinolyl, benzoxazolyl, benzothiazolyl or phenanthryl;
R² is hydrogen; and
R³ is hydrogen or halogen.

7. A compound according to Claim 1 wherein
R is CO₂R⁴;
R⁴ is hydrogen, Na, NH₄, K, Ca, Mg, trimethylsulfonium, trimethylsulfoxonium or isopropylammonium;
R¹ is Ar, (Z)ₓY-Ar or ZAr wherein Ar is an optionally substituted phenyl, pyridyl, naphthyl, indolyl, piperonyl, quinolyl, benzoxazolyl, benzothiazolyl, anthranilyl or phenanthryl wherein the optional substituents are halo, C₁-C₆-alkyl, C₂-C₆-alkenyl, C₁-C₆-haloalkyl, C₁-C₆-alkylthio, C₁-C₆-alkylsulfinyl, C₁-C₆-alkylsulfonyl, C₁-C₆-alkoxy, C₁-C₆-haloalkoxy, C₁-C₆-alkoxy-C₁-C₆-alkyl, cyano, nitro, C₁-C₆-alkoxycarbonyl, amino, C₁-C₆-alkylamino, di-C₁-C₆-alkylamino or hydroxy; Y is O, S or NH; Z is an optionally substituted C₁-C₃alkyl, C₂-C₄alkynyl, or an optionally substituted C₂-C₄ alkenyl wherein the substituents are independently C₁-C₆-alkyl and halogen; and x is 0 to 2.

8. A herbicidal composition comprising a herbicidally effective amount of a compound of Claim 1 in association with an agriculturally acceptable diluent.

## Patentansprüche

1. Verbindung der folgenden Formel: 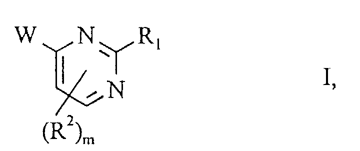 in der:
- W einen der folgenden Reste darstellt: 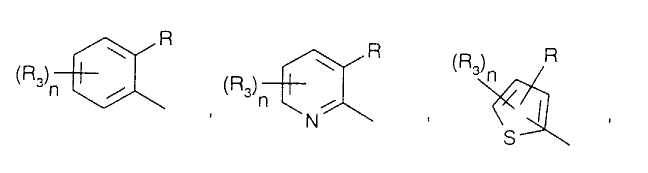 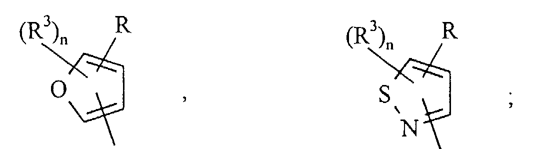 wobei:
• jeder Rest R³ unabhängig ein Wasserstoffatom, ein Halogenatom, eine C₁-C₆-Alkylgruppe, eine halogenhaltige C₁-C₆-Alkylgruppe, eine C₁-C₆-Alkoxygruppe, eine Cyanogruppe, eine C₁-C₆-Alkoxycarbonylgruppe, eine C₁-C₆-Alkylaminogruppe, eine Di-C₁-C₆-alkylarninogruppe oder -N(R⁵)-CO-R⁶ darstellt;
• n eine Zahl von 1 bis 4 ist und
• R einen der Reste CO₂R⁴, CHO, CONH-O-CH₂CO₂R⁴, COSR⁴, CO₂CHR⁵OCOR⁶ und CH=NOR⁴ darstellt;
- R¹ einen Rest Ar, (Z)ₓY-Ar oder ZAr darstellt, wobei:
• Ar ein gegebenenfalls substituierter Aryl- oder Heteroarylrest ist, der unter folgenden Gruppen ausgewählt ist: Phenyl, Pyridyl, Piperonyl, Naphthyl, Indolyl, Chinolyl, Isochinolyl, Chinoxalinyl, Chinazolinyl, Benzoxazolyl, Benzothiazolyl, Phenanthryl, Pyridyl-N-oxid, Anthranilyl, Pyrimidinyl, Pyrazinyl, Thienyl, Furyl, Pyrrolyl, Oxazolyl, Thiazolyl, Isoxazolyl, Isothiazolyl, Imidazolyl, Pyrazolyl, Oxadiazolyl und Thiodiazolyl, wobei die gegebenenfalls vorhandenen Substituenten dieser Aryl- beziehungsweise Heteroarylreste Phenoxygruppen, Halogenatome, C₁-C₆-Alkylgruppen, C₂-C₆-Alkenylgruppen, halogenhaltige C₁-C₆-Alkylgruppen, halogenhaltige C₁-C₆-Alkylthiogruppen, C₁-C₆-Alkylthiogruppen, C₁-C₆-Alkylsulfinylgruppen, C₁-C₆-Alkylsulfonylgruppen, C₁-C₆-Alkoxygruppen, halogenhaltige C₁-C₆-Alkoxygruppen, C₁-C₆-Alkoxy-C₁-C₆-alkylgruppen, Cyanogruppen, Nitrogruppen, C₁-C₆-Alkoxycarbonylgruppen, Aminogruppen, C₁-C₆-Alkylaminogruppen, Di-C₁-C₆-alkylaminogruppen oder Hydroxygruppen sind;
• Y O, S oder NH ist;
• Z eine gegebenenfalls substituierte C₁-C₃-Alkyl-, C₂-C₄-Alkinyl-oder eine gegebenenfalls substituierte C₂-C₄-Alkenylgruppe ist, deren Substituenten unabhängig voneinander unter C₁-C₆-Alkylgruppen und Halogenatomen ausgewählt sind, und
• x eine Zahl von 0 bis 2 ist;
- Rest R² unabhängig ein Wasserstoffatom, ein Halogenatom, eine C₁-C₆-Alkylgruppe, eine C₂-C₆-Alkenylgruppe, eine halogenhaltige C₁-C₆-Alkylgruppe, eine C₁-C₆-Alkylthiogruppe, eine C₁-C₆-Alkylsulfinylgruppe, eine C₁-C₆-Alkylsulfonylgruppe, eine C₁-C₆-Alkoxygruppe, eine C₁-C₆-Alkoxy-C₁-C₆-alkylgruppe, eine Cyanogruppe, eine Nitrogruppe, eine Aminogruppe, eine C₁-C₆-Alkylaminogruppe, eine Di-C₁-C₆-alkylaminogruppe, CO₂R⁴ oder eine Hydroxygruppe ist und
- m eine Zahl von 1 bis 2 ist;
• R⁴ ein Wasserstoffatom, ein Alkali- oder Erdalkalimetallkation, ein Ammoniumkation, ein substituiertes Ammoniumkation,ein Phosphoniumkation, ein C₁-C₆-Alkylphosphoniumkation, ein Tri-C₁-C₆-alkylsulfoniumkation, ein Tri-C₁-C₆-alkylsulfoxoniumkation, eine C₁-C₆-Alkylgruppe, eine C₂-C₆-Alkenylgruppe, eine halogenhaltige C₁-C₆-Alkylgruppe, eine C₁-C₆-Alkoxy-C₁-C₆-alkylgruppe oder ein gegebenenfalls substituierter Phenylrest oder ein gegebenenfalls substituierter Phenyl-C₁-C₆-alkylrest ist;
• R⁵ ein Wasserstoffatom oder eine C₁-C₆-Alkylgruppe ist und
• R⁶ eine C₁-C₆-Alkylgruppe, eine C₂-C₆-Alkenylgruppe, eine halogenhaltige C₁-C₆-Alkylgruppe, eine C₁-C₆-Alkoxy-C₁-C₆-alkylgruppe oder eine gegebenenfalls mit einer C₁-C₆-Alkylgruppe, einer halogenhaltigen C₁-C₆-Alkylgmppe, einem Halogenatom, einer C₁-C₆-Alkoxygruppe, einer C₂-C₆-Alkenylgruppe, einer Cyano- oder einer Nitrogruppe substituierte Phenyl- oder Phenyl-C₁-C₆-alkylgruppe ist;
mit der Maßgabe, daß:
(i) W nicht den Rest 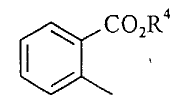 darstellt, wenn R¹ ein Phenylrest ist, und
(ii) R² keine C₁-C₆-Alkyl- oder C₂-C₆-Alkenylgruppe ist, wenn R¹ eine gegebenenfalls substituierte Phenylgruppe ist und W folgenden Rest darstellt:

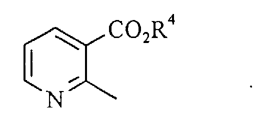

2. Verbindung nach Anspruch 1, in der:
- W folgender Rest ist: 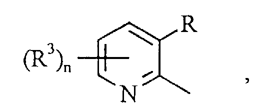 wobei R einen der Reste CO₂R⁴, CHO, CONH-O-CH₂CO₂R⁴, COSR⁴, CO₂CHR⁵OCOR⁶ und CH=NOR⁴ darstellt;
- R¹ ein Rest Ar, ZₓYAr oder ZAr ist, wobei Ar ein gegebenenfalls substituierter Phenyl-, Pyridyl-, Naphthyl-, Indolyl-, Piperonyl-, Chinolyl-, Benzoxazolyl-, Benzothiazolyl-, Anthranilyl- oder Phenanthrylrest ist;
- R² ein Wasserstoffatom, CO₂R⁴ oder eine C₁-C₆-Alkoxygruppe ist und
- R³ ein Wasserstoffatom oder ein Halogenatom ist.

3. Verbindung nach Anspruch 2, in der:
- R ein Rest CO₂CHR⁵OCOR⁶ oder CO₂R⁴ ist;
- R⁴ ein Wasserstoffatom, Na, NH₄, K, Ca, Mg, Trimethylsulfonium, Trimethylsulfoxonium oder Isopropylammonium ist und
- R¹ ein gegebenenfalls substituierter Phenyl-, Pyridyl-, Naphthyl-, Piperonyl- oder Chinolylrest oder ein Rest (Z)phenyl oder (Z)ₓOphenyl ist, wobei Z eine C₁-C₃-Alkylgruppe, eine C₂-C₄-Alkinylgruppe oder eine C₂-C₄-Alkenylgruppe darstellt und wobei x gleich 1 ist.

4. Verbindung nach Anspruch 1, in der:
- W folgender Rest ist: 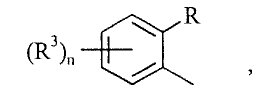 wobei R einen der Reste CO₂R⁴, CHO, CONH-O-CH₂CO₂R⁴, COSR⁴, CO₂CHR⁵OCOR⁶ und CH=NOR⁴ darstellt;
- R¹ ein Rest Ar, ZₓYAr oder ZAr ist, wobei Ar ein gegebenenfalls substituierter Phenyl-, Pyridyl-, Naphthyl-, Indolyl-, Piperonyl-, Chinolyl-, Benzoxazolyl-, Benzothiazolyl-, Anthranilyl- oder Phenanthrylrest ist;
- R² ein Wasserstoffatom, eine C₁-C₆-Alkylgruppe, CO₂R⁴ oder eine C₁-C₆-Alkoxygruppe ist und
- R³ ein Wasserstoffatom oder ein Halogenatom ist.

5. Verbindung nach Anspruch 2, in der:
- R ein Rest CO₂CHR⁵OCOR⁶ oder CO₂R⁴ ist;
- R⁴ ein Wasserstoffatom, Na, NH4, K, Ca, Mg, Trimethylsulfonium, Trimethylsulfoxonium oder Isopropylammonium ist;
- R² ein Wasserstoffatom ist und
- R¹ ein gegebenenfalls substituierter Phenyl-, Pyridyl-, Naphthyl-, Piperonyl- oder Chinolylrest oder ein Rest (Z)phenyl oder (Z)ₓOphenyl ist, wobei x gleich 1 ist.

6. Verbindung nach Anspruch 1, in der:
- W einer der folgenden Reste ist: 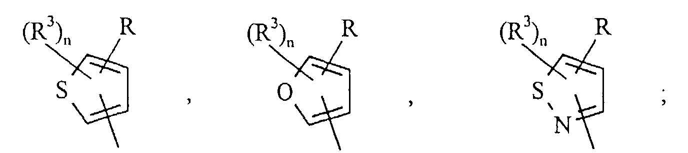 wobei R einen der Reste CO₂R⁴, CHO, CONH-O-CH₂CO₂R⁴, COSR⁴, CO₂CHR⁵OCOR⁶ und CH=NOR⁴ darstellt;
- R¹ ein Rest Ar, ZₓOAr oder ZAr ist, wobei Ar ein gegebenenfalls substituierter Phenyl-, Pyridyl-, Naphthyl-, Indolyl-, Piperonyl-, Chinolyl-, Benzoxazolyl-, Benzothiazolyl- oder Phenanthrylrest ist;
- R² ein Wasserstoffatom ist und
- R³ ein Wasserstoffatom oder ein Halogenatom ist.

7. Verbindung nach Anspruch 1, in der:
- R ein Rest CO₂R⁴ ist;
- R⁴ ein Wasserstoffatom, Na, NH4, K, Ca, Mg, Trimethylsulfonium, Trimethylsulfoxonium oder Isopropylammonium ist und
- R¹ ein Rest Ar, (Z)ₓY-Ar oder ZAr ist, wobei:
• Ar ein gegebenenfalls substituierter Phenyl-, Pyridyl-, Naphthyl-, Indolyl-, Piperonyl-, Chinolyl-, Benzoxazolyl-, Benzothiazolyl-, Anthranilyl- oder Phenanthrylrest ist, dessen gegebenenfalls vorhandene Substituenten Halogenatome, C₁-C₆-Alkylgruppen, C₂-C₆-Alkenylgruppen, halogenhaltige C₁-C₆-Alkylgruppen, C₁-C₆-Alkylthiogruppen, C₁-C₆-Alkylsulfinylgruppen, C₁-C₆-Alkylsulfonylgruppen, C₁-C₆-Alkoxygruppen, halogenhaltige C₁-C₆-Alkoxygruppen, C₁-C₆-Alkoxy-C₁-C₆-alkylgruppen, Cyanogruppen, Nitrogruppen, C₁-C₆-Alkoxycarbonylgruppen, Aminogruppen, C₁-C₆-Alkylaminogruppen, Di-C₁-C₆-alkylaminogruppen oder Hydroxygruppen sind,
• Y O, S oder NH ist;
• Z eine gegebenenfalls substituierte C₁-C₃-Alkyl-, C₂-C₄-Alkinyl- oder C₂-C₄-Alkenylgruppe ist, deren Substituenten unabhängig voneinander unter C₁-C₆-Alkylgruppen und Halogenatomen ausgewählt sind, und
• x eine Zahl von 0 bis 2 ist.

8. Herbizide Zusammensetzung, die eine herbizid wirksame Menge einer Verbindung nach Anspruch 1 in Kombination mit einem landwirtschaftlich akzeptablen Verdünnungsmittel umfasst.

## Revendications

1. Un composé de formule 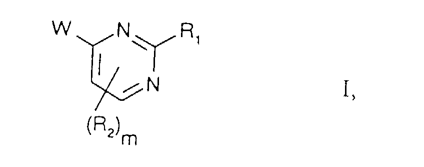 où
W signifie 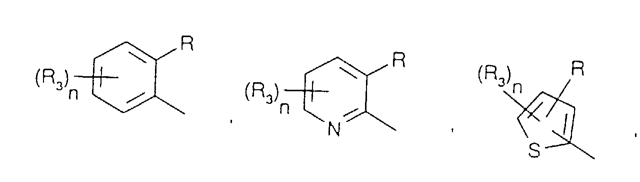 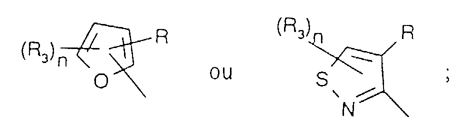
R³ signifie indépendamment l'hydrogène, un halogène, un groupe C₁-C₆-alkyle, C₁-C₆-haloalkyle, C₁-C₆-alcoxy, cyano, C₁-C₆-alcoxycarbonyle, C₁-C₆-alkylamino, di-C₁-C₆-alkylamino, ou bien -N(R⁵)-CO-R⁶;
n signifie 1 à 4;
R signifie
CO₂R⁴, CHO, CONH-O-CH₂CO₂R⁴, COSR⁴, CO₂CHR⁵OCOR⁶ ou CH=NOR⁴;
R¹ signifie Ar, (Z)ₓY-Ar, ou bien ZAr où Ar signifie un groupe aryle ou hétéroaryle éventuellement substitués choisi parmi le groupe comprenant les groupes phényle, pyridyle, pipéronyle, naphtyle, indolyle, quinolyle, isoquinolyle, quinoxalinyle, quinazolinyle, benzoxazolyle, benzothiazolyle, phénanthryle, pyridyl-N-oxyde, anthranilyle, pyrimidinyle, pyrazinyle, thiényle, furyle, pyrrolyle, oxazolyle, thiazolyle, isoxazolyle, isothiazolyle, imidazolyle, pyrazolyle, oxadiazolyle et thiodiazolyle; où les substituants éventuels signifient un groupe phénoxy, halo, un groupe C₁-C₆-alkyle, C₂-C₆-alcényle, C₁-C₆-haloalkyle, C₁-C₆-haloalkylthio, C₁-C₆-alkylthio, C₁-C₆-alkylsulfinyle, C₁-C₆-alkylsulfonyle, C₁-C₆-alcoxy, C₁-C₆-haloalcoxy, C₁-C₆-alcoxy-C₁-C₆-alkyle, cyano, nitro, C₁-C₆-alcoxycarbonyle, amino, C₁-C₆-alkylamino, di-C₁-C₆-alkylamino ou hydroxy; Y signifie O, S ou NH; Z signifie un groupe C₁-C₃-alkyle, C₂-C₄-alcynyle éventuellement substitués ou un groupe C₂-C₄-alcényle éventuellement substitué où les substituants signifient indépendamment un groupe C₁-C₆-alkyle et un halogène;
x signifie 0 à 2;
R² signifie indépendamment l'hydrogène, un halogène, un groupe C₁-C₆-alkyle, C₂-C₆-alcényle, C₁-C₆-haloalkyle, C₁-C₆-alkylthio, C₁-C₆-alkylsulfinyle, C₁-C₆-alkylsulfonyle, C₁-C₆-alcoxy, C₁-C₆-alcoxy-C₁-C₆-alkyle, cyano, nitro, amino, C₁-C₆-alkylamino, di-C₁-C₆-alkylamino, CO₂R⁴ ou hydroxy;
m signifie 1 à 2;
R⁴ signifie l'hydrogène, un cation de métal alcalin ou de métal alcalino-terreux, un cation ammonium, un cation ammonium substitué, un cation phosphonium, un cation C₁-C₆-alkylphosphonium, un cation tri- C₁-C₆-alkylsulfonium, un cation tri-C₁-C₆-alkylsulfoxonium, un groupe C₁-C₆-alkyle, C₂-C₆-alcényle, C₁-C₆-haloalkyle, C₁-C₆-alcoxy-C₁-C₆-alkyle, un groupe phényle éventuellement substitué ou phényl-C₁-C₆-alkyle éventuellement substitué;
R⁵ signifie l'hydrogène ou un groupe C₁-C₆-alkyle; et
R⁶ signifie un groupe C₁-C₆-alkyle, C₂-C₆-alcényle, C₁-C₆-haloalkyle, C₁-C₆-alcoxy-C₁-C₆-alkyle, phényle éventuellement substitué ou phényl-C₁-C₆-alkyle éventuellement substitué, où les substituants éventuels signifient le groupe C₁-C₆-alkyle, C₁-C₆-haloalkyle, un halogène, C₁-C₆-alcoxy, C₂-C₆-alcényle, cyano et nitro et avec la condition que (i) lorsque R¹ signifie un groupe phényle; W ne signifie pas 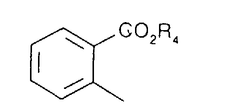 et (ii) lorsque R¹ signifie un groupe phényle éventuellement substitué et W signifie 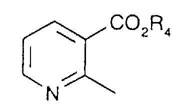 R² ne signifie pas un groupe C₁-C₆-alkyle ou C₂-C₆-alcényle.

2. Un composé selon la revendication 1, où W signifie 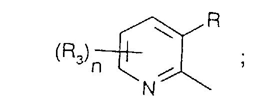
R signifie
CO₂R⁴; CHO; CONH-O-CH₂CO₂R⁴; COSR⁴; CO₂CHR⁵OCOR⁶ ou CH=NOR⁴;
R¹ signifie Ar, ZₓYAr ou ZAr où Ar signifie un groupe phényle, pyridyle, naphtyle, indolyle, pipéronyle, quinolyle, benzoxazolyle, benzothiazolyle, anthranilyle ou phénanthryle éventuellement substitués;
R² signifie l'hydrogène, CO₂R⁴ ou C₁-C₆-alcoxy; et
R³ signifie l'hydrogène ou un halogène.

3. Un composé selon la revendication 2, où R signifie CO₂CHR⁵OCOR⁶ ou CO₂R⁴; R⁴ signifie l'hydrogène, Na, NH4, K, Ca, Mg, triméthylsulfonium, triméthylsulfoxonium ou isopropylammonium, R¹ signifie un groupe phényle, pyridyle, naphtyle, pipéronyle, quinolyle, (Z)phényle,ou (Z)ₓOphényle éventuellement substitués; Z signifie un groupe C₁-C₃-alkyle , C₂-C₄-alcynyle ou C₂-C₄-alcényle et x signifie 1.

4. Un composé selon la revendication 1, où W signifie 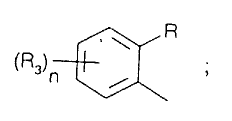
R signifie
CO₂R⁴; CHO; CONH-O-CH₂CO₂R⁴; COSR⁴; CO₂CHR⁵OCOR⁶ ou CH=NOR⁴;
R¹ signifie Ar, ZₓYAr ou ZAr où Ar signifie un groupe phényle, pyridyle, naphtyle, indolyle, pipéronyle, quinolyle, benzoxazolyle, benzothiazolyle, anthranilyle ou phénanthryle éventuellement substitués;
R² signifie l'hydrogène, un groupe C₁-C₆-alkyle, CO₂R⁴ ou C₁-C₆-alcoxy; et
R³ signifie l'hydrogène ou un halogène.

5. Un composé selon la revendication 2, où R signifie CO₂CHR⁵OCOR⁶ ou CO₂R⁴; R⁴ signifie l'hydrogène, Na, NH4, K, Ca, Mg, triméthylsulfonium, triméthylsulfoxonium ou isopropylammonium; R² signifie l'hydrogène et R¹ signifie un groupe phényle, pyridyle, naphtyle, pipéronyle, quinolyle, (Z)phényle, ou (Z)ₓOphényle éventuellement substitués et x signifie 1.

6. Un composé selon la revendication 1, où W signifie 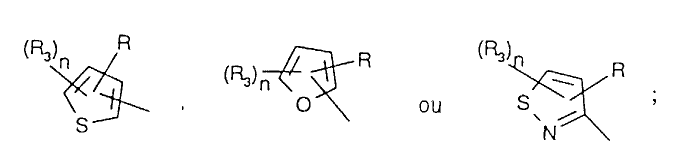
R signifie
CO₂R⁴; CHO; CONH-O-CH₂CO₂R⁴; COSR⁴; CO₂CHR⁵OCOR⁶OU CH=NOR⁴;
R¹ signifie Ar, ZₓOAr ou ZAr où Ar signifie un groupe phényle, pyridyle, naphtyle, indolyle, pipéronyle, quinolyle, benzoxazolyle, benzothiazolyle ou phénanthryle éventuellement substitués;
R² signifie l'hydrogène; et
R³ signifie l'hydrogène ou un halogène.

7. Un composé selon la revendication 1, où
R signifie CO₂R⁴;
R⁴ signifie l'hydrogène, Na, NH₄, K, Ca, Mg, triméthylsulfonium, triméthylsulfoxonium ou isopropylammonium;
R¹ signifie Ar, (Z)ₓY-Ar ou ZAr où Ar signifie un groupe phényle, pyridyle, naphtyle, indolyle, pipéronyle, quinolyle, benzoxazolyle, benzothiazolyle, anthranilyle ou phénanthryle éventuellement substitués où les substituants éventuels signifient halo, C₁-C₆-alkyle , C₂-C₆-alcényle, C₁-C₆-haloalkyle, C₁-C₆-alkylthio, C₁-C₆-alkylsulfinyle, C₁-C₆-alkylsulfonyle, C₁-C₆-alcoxy, C₁-C₆-haloalcoxy, C₁-C₆-alcoxy-C₁-C₆-alkyle, cyano, nitro, C₁-C₆-alcoxycarbonyle, amino, C₁-C₆-alkylamino, di-C₁-C₆-alkylamino ou hydroxy; Y signifie O, S ou NH; Z signifie un groupe C₁-C₃-alkyle, C₂-C₄-alcynyle éventuellement substitués ou un groupe C₂-C₄-alcényle éventuellement substitué où les substituants signifient indépendamment un groupe C₁-C₆-alkyle et un halogène; et x signifie 0 à 2.

8. Une composition herbicide comprenant une quantité efficace du point de vue herbicide d'un composé selon la revendication 1 en association avec un diluant acceptable en agriculture.
